(19) Europäisches Patentamt
European Patent Office
Office européen des brevets

(11) Publication number: **0 250 096 A1**

# (12) EUROPEAN PATENT APPLICATION

(21) Application number: **87304458.0**

(22) Date of filing: **20.05.87**

(51) Int. Cl.[4]: **C07D 413/04**, C07K 5/06, A61K 31/42, A61K 37/02

(30) Priority: **21.05.86 JP 116674/86**

(43) Date of publication of application: **23.12.87 Bulletin 87/52**

(84) Designated Contracting States: **AT BE CH DE ES FR GB GR IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Masuya, Hirotomo**
**1-19, Matsuodai 4-chome Inagawa-cho**
**Kawabe-gun Hyogo 666-02(JP)**

(74) Representative: **Lewin, John Harvey et al**
**Elkington and Fife High Holborn House 52/54**
**High Holborn**
**London WC1V 6SH(GB)**

## (54) Isoxazolone derivatives and production thereof.

(57) The present invention related to a compound of the formula :

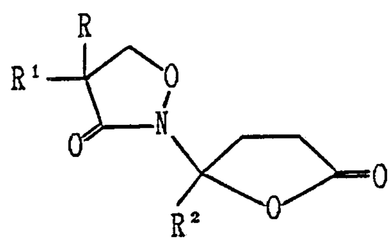

(I)

wherein $R^1$ is hydrogen, isonitrile or an organic residue bonded through nitrogen ; R is hydrogen, alkyl which may be substituted, alkylthio in which sulfur atom may be oxidized or azido, or forms a double bond together with the adjacent carbon atom; provided that when $R^1$ is an organic residue bonded through nitrogen, R is not hydrogen; $R^2$ is carboxyl or a group derivable therefrom, and a salt thereof, and a method for producing the same.

The Compound (I) of the present invention has, among others, an excellent anitimicrobial activity and is useful as an antimicrobial agent, etc.

EP 0 250 096 A1

# Isoxazolone Derivatives and Production Thereof

This invention relates to novel 2-(3-oxo-2-isoxazolidinyl)-5-oxo-2-tetrahydrofurancarboxylic acid derivatives exhibiting excellent antimicrobial and $\beta$-lactamase inhibitory activities.

Recently, a novel antibiotic TAN-588 (hereinafter referred to in some instances briefly as "TAN-588") exhibiting antimicrobial activity against gram-positive and gram-negative bacteria has been harvested from new species of microorganisms belonging to the genera Empedobacter and Lysobacter as isolated from soil. The chemical structure of the said antibiotic TAN-588 has been under elucidation, and as a result, it has been found that the antibiotic TAN-588 has a peculiar skeleton consisting of a 3-oxoisoxazolidine ring having 5-oxo-2-tetrahydrofurancarboxylic acid bonded at its nitrogen atom.

Heretofore, there has been reported a synthesis of a compound consisting of a 3-oxoisoxazolidine ring having a 1-methylacetic acid group introduced at its nitrogen atom [Tsuji and Yamana; Heterocycles, 8 p.153, (1977)]. However, it has been reported that no antimicrobial activity was observed in the compound having the said 1-methylacetic acid group.

The present invention is directed to the production of novel and useful 2-(3-oxo-2-isoxazolidinyl)-5-oxotetrahydrofurancarboxylic acid derivatives having antimicrobial activities.

The present invention is to provide compounds representable by the formula;

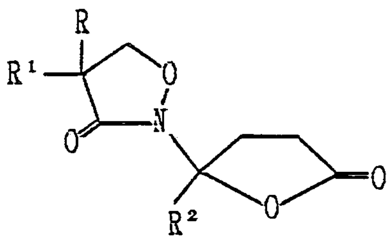

(I)

[wherein $R^1$ stands for hydrogen, isonitrile or an organic residue bonded through nitrogen; R stands for hydrogen, alkyl which may be substituted, alkylthio in which sulfur atom may be oxidized or azido, or forms a double bond together with the adjacent carbon atom; provided that when $R^1$ is an organic residue bonded through nitrogen, R is not hydrogen; and $R^2$ stands for carboxy or a group derivable therefrom] or salts thereof.

Referring to the compounds representable by the above formula (I), more detailed description is given as follows. Among the groups in the following description, those bearing the asterisk * are optionally substituted ones.

Examples of the organic residue bonded through nitrogen as represented by $R^1$ include acylamino, amino substituted through carbon, alkenylamino, thioamino, silylamino, phospho amino and a group represented by the formula -CO-CO-NH-.

The acyl in the above acylamino is exemplified by known acyl groups, such as those being substituted at the 6-amino group of penicillin derivatives and those being substituted at the 7-amino group of cephalosporin derivatives.

Examples of the said acylamino group include those of the formula:

$$R^3\text{-CO-}\underset{\displaystyle R^4}{\underset{|}{N}}\text{-}$$

[wherein $R^3$ stands for hydrogen, alkyl*, alkenyl*, cycloalkyl*, aryl*, heterolcyclic ring*, alkoxy* or aryloxy*; $R^4$ stands for hydrogen or alkyl*, and $R^4$ also cooperates with $R^3$ to form a ring*]; those of the formula $R^5$

$$\text{-NH-}\underset{\displaystyle R^6}{\underset{|}{\text{CH}}}\text{-CO-NH-}$$

[wherein $R^5$ stands for hydrogen, amino acid residue*, amino-protecting group or a group represented by the formula $R^7$ -(CH )n-C(=Z)-{wherein $R^7$ stands for heterocyclic ring*, alkoxy* or amino*; n denotes 0, 1 or 2; a nd Z stands for O or S}; $R^6$ stands for alkyl* aryl*. cycloalkenyl* or heterocyclic ring*]; those of the formula $R^8$-$R^9$-CO-NH-[wherein $R^8$ stands for a group representable by the formula $R^{10}$

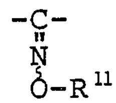

{wherein $R^{10}$ stands for alkyl*, heterocyclic ring* or aryl*, $R^{11}$ stands for hydrogen, alkyl*, alkenyl*, cycloalkyl*, heterocyclic ring* or a group representable by the formula $-R^{12}-R^{13}$ (wherein $R^{12}$ stands for alkylene*, cycloalkylene or alkenylene and $R^{13}$ stands for aryl*, carboxy* or its ester or mono-or dialkylamido)} , and $R^9$ stands for a chemical bond or a group representable by the formula

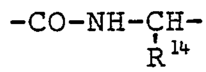

(wherein $R^{14}$ stands for alkyl*, aryl* or heterocyclic ring*)]; those of the formula $R^{15}$

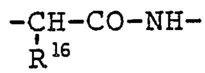

[$R^{15}$ stands for aryl*, heterocyclic ring* or cycloalkenyl* and $R^{16}$ stands for hydroxy, sulfamoyl, sulfo, sulfoxy or acyloxy*]; those of the formula $R^{17}-R^{18}-CH_2-CO-NH-$[wherein $R^{17}$ stands for alkyl*, cyano, aryl*, aryloxy*, alkenylene*, heterocyclic ring*, amino* or a group represented by the formula $R^{17'}-C(=S)-$(wherein $R^{17'}$ stands for alkoxy) and $R^{18}$ stands for a chemical bond or -S-]; and those of the formula

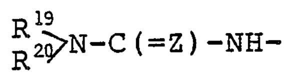

[wherein $R^{19}$ and $R^{20}$ independently stand for hydrogen, alkyl*, aryl*, heterocyclic ring* or cycloalkyl, and Z stands for O or S].

The formula $R^{10}$

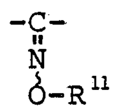

in the group $R^8$ represents the <u>syn</u> isomer represented by the formula

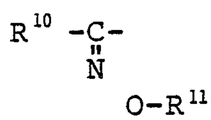

and the the <u>anti</u> isomer represented by the formula

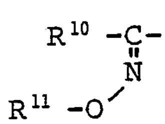

or a mixture thereof.

Examples of the amino being substituted through carbon which exemplifies the organic residue bonded through nitrogen as represented by the above $R^1$ include groups of the formula $R^{21}-NH-$[wherein $R^{21}$ stands for alkyl*, aryl*, alkenyl* or heterocyclic ring*], those of the formula

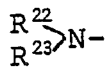

[wherein $R^{22}$ and $R^{23}$ independently stand for alkyl*, aryl* or alkenyl*, and they cooperate, depending on cases, with the adjacent nitrogen atom to form a heterocyclic ring], and those of the formula

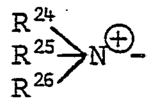

[wherein $R^{24}$, $R^{25}$ and $R^{26}$ independently stand for alkyl*, aryl* or alkenyl, and they cooperate, depending on cases, with the adjacent nitrogen atom to form a heterocyclic ring*].

Examples of the alkenylamino which exemplifies the group represented by the above $R^1$ include groups of the formula

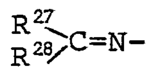

[wherein $R^{27}$ and $R^{28}$ independently stand for hydrogen, alkyl*, aryl*, cycloalkyl, amino* or heterocyclic ring*, and they cooperate, depending on cases, with the adjacent carbon atom to form cycloalkyl* or heterocyclic ring*].

Examples of the thioamino which exemplifies the group represented by the above $R^1$ include groups of the formula $R^{29}$-$SO_n$ -NH-[wherein $R^{29}$ stands for alkyl* or aryl*, and n denotes 0, 1 or 2].

Examples of the silylamino which exemplifies the group represented by the above $R^1$ include groups of the formula

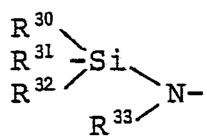

[wherein $R^{30}$, $R^{31}$ and $R^{32}$ independently stand for alkyl* or aryl* and they form, depending on case, form a cyclic group, and $R^{33}$ stands for hydrogen or silyl*].

Examples of the phosphoamino which exemplifies the group represented by the above $R^1$ include groups of the formula

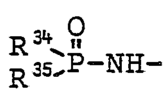

[wherein $R^{34}$ and $R^{35}$ independently stand for alkyl*, aryl*, alkoxy* or aryloxy, and they form, depending on cases, a heterocyclic ring*].

Examples of the group represented by the above $R^1$ include groups of the formula $R^{36}$-CO-CO-NH-[wherein $R^{36}$ stands for hydrogen, alkyl*, alkoxy*, aryl*, aryloxy*, heterocyclic ring* or amino*].

In the above formulae, the organic residue bonded through nitrogen as represented by $R^1$ is preferably those of a molecular weight of up to 500.

As the optionally substituted alkyl as represented by R are mentioned straight-chain or branched lower alkyl groups, preferably those of 1 to 6 carbon atoms, which are exemplified by methyl, ethyl, propyl, i-propyl, butyl, i-butyl, sec-butyl, t-butyl, pentyl and hexyl. These alkyl groups may be substituted, and the substituent is exemplified by hydroxy (primary, secondary, tertiary), substituted hydroxycarbonyl (benzhydolyloxycarbonyl, benzyloxycarbonyl, etc.), substituted carbonyloxy (lower alkanoyloxy, aminocarbonyloxy, acylaminocarbonyloxy, etc.), lower($C_{1-4}$)alkyl sulfonyloxy, azido, etc.

Examples of the alkylthio in which sulfur atom is oxidized, as represented by R, include a lower($C_{1-4}$)-alkylthio. The sulfur atom may have 1 or 2 oxygen atoms. These alkylthio groups are exemplified by methylthio, ethylthio, methylsulfinyl, methylsulfonyl, etc.

When R cooperates with the adjacent carbon atom to form a double bond, formation of the double bond with the adjacent carbon atom on the isoxazole ring (endo double bond) and formation of the double bond with the adjacent carbon atom on $R^1$ (exo double bond) are mentioned.

Examples of the group derivable from the carboxy, as represented by $R^2$ in the above formulae, include groups of the formula $-COOR^{37}$ [wherein $R^{37}$ stands for alkyl*, alkenyl*. aryl*. cycloalkyl*, heterocyclic ring* or silyl*] and groups of the formula

$$-CO-N\begin{matrix}\diagup R^{38} \\ \diagdown R^{39}\end{matrix}$$

[wherein $R^{38}$ and $R^{39}$ independently stand for hydrogen, alkyl*, aryl*, cycloalkyl*, alkenyl* or heterocyclic ring*, and they cooperate with the adjacent nitrogen atom to form a heterocyclic ring].

In the above formulae, the group derivable from carboxy, as represented by $R^2$, is preferably those of a molecular weight of up to 500.

In the above formulae, the alkyl groups as represented by $R^3$, $R^4$, $R^6$, $R^{10}$ , $R^{11}$ , $R^{14}$ , $R^{17}$ , and $R^{19-39}$ are preferably those having 1 to 6 carbon atoms, which are exemplified by methyl, ethyl, n-propyl, isopropyl, n-butyl, isobutyl, sec-butyl, t-butyl, 1,1-dimethylpropyl, n-pentyl, isopentyl, n-hexyl, isohexyl, etc.

The substituent which the said alkyl group may have includes, for example, halogen, oxo, thioxo, nitro, amino (optioanlly substituted by alkyl, alkenyl, cycloalkyl, aryl, acyl, carbamoyl, N-sulfocarbamoyl), sulfo, cyano, hydroxy, carboxy (optionally esterified with alkyl), cycloalkyl, cycloalkenyl, alkoxy (optionally substituted by amino, hydroxy, carboxy, halogen, aryl, cycloalkyl, alkoxy), aryl (optioanlly substituted by halogen, alkyl, alkoxy, alkylamino, amino, carbamoyl, sulfo, alkylsulfonyl, cyano, hydroxy, carboxy, nitro, acyloxy, aralkyloxy, sulfoxy), arylcarbonyl optionally having similar substituents to those of the above aryl, aryloxy optionally having similar substituents to those of the above aryl, heterocyclic ring (optionally substituted by nitro, oxo, aryl, alkenylene, halogenoalkyl, alkylsulfonyl, alkyl, alkoxy, alkylamino, amino, halogen, carbamoyl, hydroxy, cyano, carboxy, sulfo), acyl (optionally substituted by arylcarbonylhydrazino optionally substituted by hydroxy, halogen, amino, nitro), acyloxy, alkoxycarbonyl, alkoxycarbonyloxy (optionally substituted by halogen), acyloxyethoxy, aralkyl (optioanlly substituted by alkyl, alkoxy, halogen, amino hydroxy, nitro, cyano, carbamoyl, sulfamoyl), aralkyloxy (optionally substituted by acyloxy, alkyl, alkoxy, halogen, amino, hydroxy, nitro, cyano, carbamoyl, sulfamoyl), hydroxysulfonyloxy alkylsulfonyloxy, arylsulfonyloxy, alkylsulfonyl, aminosulfonyl, alkylsulfinyl, arylsulfonyl, alkylsulfinyl, alkylthio (optionally substituted by cyano, halogen, carboxy, alkylamino, imino, carbamoyl, acylamino), arylthio, heterocyclic ring-thio (optionally substituted by cyano, hydroxy, amino, alkylamino, alkyl, halogen, oxo), heterocyclic ring (optionally substituted by cyano, hydroxy, amino, alkylamino, alkyl, halogen, oxo)-alkyl-thio, iminomethylamino, iminoethylamino, silyl (optioannly substituted by alkyl, aryl), alkyloxycarbonyl, arylcarbonyl (optionally substituted by acyloxy, halogen, amino, hydroxy, alkoxy, sulfamoyl), phthalimide,succinimide, dialkylamino, dialkylaminocarbonyl, arylcarbonylamino, carbamoyl, carbamoyloxy, N-sulfocarbamoyloxy, alkylcarbonylcarbamoyloxy (optionally substituted by halogen), alkoxyimino, a group representable by the formula

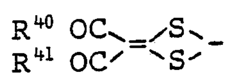

(wherein $R^{40}$ , $R^{41}$ independently stand for hydroxyl group or amino group), etc.

In the above formulae, the alkylene as represented by R is preferably those having 1 to 6 carbon atoms and exemplified by methylene, ethylene, trimethylene, tetramethylene, pentamethylene, hexamethylene, etc.

The substituent which the said alkylene group may optionally have is exemplified by halogen, amino, hydroxy, alkoxy, carbamoyl, cyano, nitro, etc.

In the above formulae, the cycloalkyl in, or the cycloalkyl formed by, $R^3$, $R^{11}$ , $R^{19}$ , $R^{20}$ , $R^{27}$ , $R^{28}$ , $R^{37}$ , $R^{38}$ and $R^{39}$ , and the cycloalkyl in cycloalkyloxy are preferably those of 3 to 8 carbon atoms, which are exemplified by cyclopropyl, cyclobutyl, cyclopentyl, cyclohexyl, cycloheptyl, cyclooctyl, etc.

The substituent which the said cycloalkyl group may optionally have is exemplified by halogen, nitro, amino, hydroxy, sulfo, cyano, carboxy, oxo, thioxo, etc.

The cycloalkylene as represented by $R^{12}$ includes, for example, those consisting of the above cycloalkyl having one more chemical bond.

In the above formulae, the ary including that in aryl, arylcarbonyl, aryloxycarbonyl or aryloxy, as represented by $R^3$, $R^6$, $R^{10}$ , $R^{13-17}$ , $R^{19-32}$ or $R^{34-39}$ is exemplified by phenyl, naphthyl, biphenyl, anthryl, indenyl, etc.

The substituent which the said aryl group may optionally have is exemplified by halogen, nitro, cyano, amino (optionally substituted by alkyl, alkenyl, cycloalkyl or aryl), sulfo, mercapto, hydroxy, carboxy, acyl, sulfoxy, sulfamoyl, carbamoyl, alkyl (optioanlly substituted by amino, halogen, hydroxy, cyano or carboxy), alkoxy, aralkyloxy, alkyl sulfonamide, methylenedioxy, alkyl sulfonyl, alkyl sulfonyl amino, etc. The aryl group may form, together with cycloalkyl, a condensed ring (e.g. tetrahydronaphthyl, indanyl, acenaphthyl, etc.)

In the above formulae, the alkoxy as represented by $R^3$, $R^7$, $R^{17'}$or $R^{34-36}$ is preferably those having 1 to 6 carbon atoms and exemplified by methoxy, ethoxy, n-propoxy, i-propoxy, n-butoxy, i-butoxy, t-butoxy, n-pentyloxy, n-hexyloxy, etc.

The substituent which the said alkoxy group may optionally have is exemplified by halogen, nitro, amino, hydroxy, sulfo, cyano, carboxy, aryl (optionally substituted by nitro, amino, hydroxy, alkyl or alkoxy), silyl (optionally substituted by alkyl, aryl or aralkyl), etc.

In the above forumulae, the alkenyl or alkenylene as represented by $R^3$, $R^{11}$ , $R^{21-26}$ or $R^{37-39}$ is preferably those having 1 to 6 carbon atoms and exemplified by methylene, vinyl, allyl, isopropenyl, 1-propenyl, 2-butenyl, 3-methyl-3-butenyl, 1,3-pentadienyl, 4-pentaenyl, 1,3-hexadienyl, ethylidene, propylidene, isopropylidene, butylidene, etc.

The substituent which the said alkenyl group may optionally have is exemplified by halogen, nitro, amino (optionally substituted by acyl), sulfo, cyano, hydroxy, carboxy, carbamoyl, sulfamoyl, aryl, acyl, etc.

In the above formula, the alkenylene as represented by $R^{12}$ or $R^{17}$ is preferably those having 2 to 6 carbon atoms and exemplified by vinylene, 1-propenylene, 2-butenylene, 2-pentenylene, 1,3-hexadienylene, etc.

The substituent which the said alkenylene group may optionally have is exemplified by halogen, cyano, carbamoyl, etc.

In the above formula, the cycloalkenyl as represented by $R^6$ or $R^{15}$ is preferably those having 3 to 8 carbon atoms and exemplified by 1-cyclopropenyl, 1-cyclobutenyl, 1-cyclopentenyl, 2-cyclopentenyl, 3-cyclopentenyl, 1-cyclohexenyl, 2-cyclohexenyl, 3-cyclohexenyl, 1-cycloheptenyl, 1,4-cyclohexadienyl, etc.

The substituent which the said cycloalkenyl group may optionally have is exemplified by halogen, nitro, amino, sulfo, sulfamoyl, etc.

In the above formula, heterocyclic ring as represented by $R^3$, $R^6$, $R^7$, $R^{10}$ , $R^{14}$ , $R^{15}$ , $R^{17}$ , $R^{19-28}$ or $R^{34-39}$ or heterocyclic ring formed by these groups is exemplified by 5-7 membered heterocyclic ring group containing one sulfur atom, nitrogen atom or oxygen atom, 5-6 membered heterocyclic ring group containing 2-4 nitrogen atoms, or 5-6 membered heterocyclic ring group containing 1-2 nitrogen atoms and one sulfur atom or oxygen atom, and these heterocyclic ring group may be condensed with a six-membered ring group containing two or less nitrogen atoms or a five-membered ring group containing benzene ring or one sulfur atom.

As specific examples of the said heterocyclic ring group are mentioned 2-pyridyl, 3-pyridyl, 4-pyridyl, pyrimidinyl, pyrazinyl, pyridazinyl, piperazinyl, piperidyl, pyrazolyl, pyranyl, thiopyranyl, pyrimidinyl, imidazolyl, thiazolyl, isothiazolyl, oxazolyl, isoxazolyl, pyrido[2,3-d]pyrimidyl, benzopyranyl, 1,8-naphthylidyl, 1,5-naphthylidyl, 1,6-naphthylidyl, 1,7-naphthylidyl, 2,7-naphthylidyl, 2,6-naphthylidyl, quniolyl, thieno[2,3-b]-pyridyl, tetrazolyl, thiadiazolyl, oxadiazolyl, triazinyl, triazolyl, thienyl, pyrrolyl, furyl, pyrrolidinyl, imidazolidinyl, dithietane, tetrahydropyranyl, tetrahydrofuranyl, benzothienyl, pyranyl, hexahydro-1H-azepinyl, indolyl, isoindolidinyl, chromanyl, etc.

The substituent which the said heterocyclic ring may optionally have is exemplified by amino(optionally substituted by halogen-substituted alkyl acyl, phenyl or alkyl), halogen, nitro, sulfo, cyano, hydroxy, carboxy, oxo, thioxo, $C_{1-10}$ alkyl [optionally substituted by aryl, halogen, amino, hydroxy, carboxy, alkoxy, alkylsulfonyl, dialkylamino, or phosphoric acid(optionally substituted by alkyl)],cycloalkyl, alkoxy(optionally substituted by halogen or hydroxy), $C_{1-4}$acyl, aryl(optionally substituted by halogen, nitro, alkyl, alkoxy, amino, sulfo, hydroxy or cyano), oxo, thioxo, amino acid residue - thio (examples of the amino acid residue are mentioned those similar to those described later), $C_{1-10}$alkylthio[optionally substituted by aryl, halogen, amino, hydroxy, carboxy, alkoxy, alkylsulfonyl, dialkylamino, or phosphoric acid(optionally substituted by alkyl)], heterocyclic ring(optionally substituted by alkyl, alkoxy, halogen, nitro, cyano, carboxy, formyl or alkylsulfonyl) or a group representable by the formula $R^{42}$-CH=N-[wherein $R^{42}$ stands for heterocyclic ring-(optionally substituted by alkyl, alkoxy, halogen, nitro, cyano, hydroxy, carboxy, formyl or alkylsulfonyl)].

In the above formula, the ring which $R^4$ forms with $R^3$, is exemplified by those formed together with phthaloyl, succinyl, maleoyl, citraconoyl, glutaryl, adipoyl, etc., and the ring formed is exemplified by 2,2-dimethyl-5-oxo-4-phenyl-imidazolidine. The substituent which the said cyclic group may optionally have is exemplified by halogen, nitro, amino, hydroxyl, sulfo, cyano, carboxy, etc.

In the above formula, the acyl in the acyloxy representable by $R^{16}$ is preferably those having 1 to 4 carbon atoms, as exemplified by formyl, acetyl, propionyl, butyryl, isobutyryl, etc., and, as its substituent are exemplified by alkyl(optionally substituted by amino, halogen, cyano, alkoxy, carboxy or hydroxy), etc.

In the above formula, the amino acid residue as represented by $R^5$ is exemplified by glycyl, alanyl, valyl, leucyl, isoleucyl, seryl, threonyl, cysteinyl, cystyl, methionyl, α-or β-asparagyl, α-or γ-glutamyl, lysyl, arginyl, phenylglycyl, tyrosyl, histidyl, tryptophanyl, prolyl, etc.

The substituent which the said amino acid residue may optionally have is exemplified by halogen, hydroxy, sulfo, carboxy, cyano, alkylamino, aralkyloxycarbonyl, aralkyloxy, guanidino, etc.

As the amino-protecting group as represented by Rs use is conveniently made of, for example, those usable for this purpose in the fields of β-lactam and peptide synthesis. Their examples include, for example, aromatic acyl groups such as phthaloyl, 4-nitrobenzoyl, 4-tert-butylbenzoyl, 4-tert-butylbenzenesulfonyl, benzenesulfonyl,toluenesulfonyl, etc.; aliphatic acyl groups such as formyl, acetyl, propionyl, monochloroacetyl, dichloroacetyl, trichloroacetyl, methanesulfonyl, ethanesulfonyl, trifluoroacetyl, malonyl, succinyl, etc.; esterified carboxyl groups such as methoxycarbonyl, ethoxycarbonyl, t-butoxycarbonyl, isopropoxycarbonyl, 2-cyanoethoxycarbonyl, 2,2,2-trichloroethoxycarbonyl, benzyloxycarbonyl, 4-nitrobenzyloxycarbonyl, diphenylmethyloxycarbonyl, methoxymethyloxycarbonyl, acetylmethyloxycarbonyl, isobornyloxycarbonyl, phenyloxycarbonyl, etc.; methylene groups such as (hexahydro-1H-azepin-1-yl)methylene; sulfonyl groups such as 2-amino-2-carboxyethylsulfonyl, etc.; and amino-protecting groups other than acyl groups, such as trityl, 2-nitrophenylthio, benzylidene, 4-nitrobenzylidene, di-or trialkylsilyl, benzyl, 4-nitrobenzyl, etc. Selection of the said protective groups is not limited in this invention, but among others, monochloroacetyl, benzylcarbonyl, 4-methoxybenzyloxycarbonyl and 4-nitrobenzyloxycarbonyl are preferable.

In the above formulae, the substituent group in the carboxy group which may have a substituent as represented by $R^{13}$ includes, for example, alkyl (which may optionally have as a substituent halogen, cyano or hydroxy), aryl (which may optionally have as a substituent alkyl, alkoxy, halogen, hydroxy, acyloxy, sulfo, cyano or sulfamoyl), silyl (which may optionally have as a substituent alkyl, aryl or aralkyl), heterocyclic rings (which may optioanlly have as a substituent amino, alkylamino, sulfamoyl, carbamoyl, halogen, cyano or nitro), etc.

In the above formulae, the ester group in the ester of carboxyl as represented by $R^{13}$ is exemplified by alkyl esters of 1 to 6 carbon atoms, and specific examples thereof include methyl ester, ethyl ester, propyl ester, n-butyl ester, isobutyl ester, tert-butyl ester, etc.

In the above formulae, the substituent group in the amino which may optioanlly have a substituent as represented by $R^7$ , $R^{17}$ , $R^{27}$ , $R^{28}$ and $R^{36}$ includes, for example, amidine, iminomethyl, imino(aryl-substituted)methyl, guanidylcarbonyl, heterocyclic ring* (which may have substituents similar to those mentioned above for the heterocyclic rings), imino (optionally substituted by heterocyclic ring)methyl, arylcarbonyl, hydroxyalkyl, alkyl, etc.

In the above formulae, the substituent group in the silyl group which may optionally have a substituent as represented by $R^{33}$ and $R^{37}$ includes, for example, alkyl, aryl, aralkyl, etc.

The above $R^{30}$ , $R^{31}$ and $R^{32}$ , together with $R^{33}$ , may form a cyclic group, and its examples include 2,5-disilylazacyclopentyl, etc. which may have optionally a substituent such as alkyl, aryl, etc.

The halogen as the above substituent group is exemplified by chlorine, bromine, fluorine and iodine.

The alkyl in the description of the above substituent groups is preferably those of 1 to 10, more preferably those of 1 to 6 or still more preferably those of 1 to 4 carbon atoms, and their examples include methyl, ethyl, n-propyl, i-propyl, n-butyl, i-butyl, t-butyl, sec-butyl, n-pentyl, isopentyl, n-hexyl, isohexyl, heptyl, octyl, nonyl, decyl, etc.

The cycloalkyl in the description of the above substituent groups is preferably those of 3 to 6 carbon atoms as exemplified by cyclopropyl, cyclobutyl, cyclopentyl, cyclohexyl, etc.

The alkoxy in the description of the above substituent groups is preferably those of 1 to 4 carbon atoms, as exemplified by methoxy, ethoxy, n-propoxy, i-propoxy, n-butoxy, i-butoxy, t-butoxy, etc.

The aryl in the description of the above substituent groups is exemplified by phenyl, naphthyl, etc.

The heterocyclic ring in the description of the above substituent groups includes those similar to the heterocyclic rings mentioned above for $R^3$, etc.

The acyl in the description of the above substituent groups is preferably those of 1 to 6, more preferably 1 to 4 carbon atoms, as exemplified by formyl, acetyl, propionyl, butyryl, isobutyryl, valeryl, isovaleryl, pivaloyl, hexanoyl, etc.

The aralkyl in the description of the above substituent groups is exemplified by benzyl, phenethyl, phenyl-propyl, etc.

The alkenyl in the description of the above substituent groups includes those similar to the alkenyl mentioned above for $R^3$ etc.

The amino acid residue in the description of the above substituent groups includes those similar to the amino acid residues mentioned above for $R^5$.

The number of the substituent groups in each of the above groups is preferably 1 to 3.

More preferable examples of the acyl group in the above acylamino as represented by R include those representable by the formula

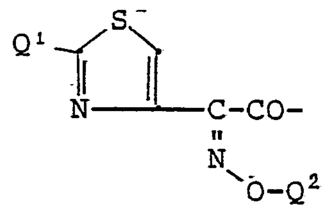

[wherein Q stands for amino or a protected amino group, and $Q^2$ stands for hydrogen, alkyl, alkenyl, a group $-CH_2COOQ^3$ or a group

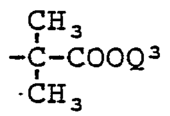

(wherein $Q^3$ stands for hydrogen or alkyl*)] As "alkyl", "alkyl*", "alkenyl" and "protective group" in the protected amino group in the above formula are mentioned those similar to those described above.

In the above acyl groups, specific examples of the acylamino group as represented by the formula

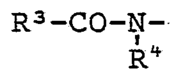

include, for example, 3-(2,6-dichlorophenyl)-5-methylisoxazol-4-yl-carbonylamino, 4-ethyl-2,3-dioxo-1-piperazinocarbonylamino, 3-phenyl-5-methylisoxazol-4-yl-carbonylamino, 3-(2-chlorophenyl)-5-methylisoxazol-4-yl-carbonylamino, 3-(2-chloro-6-fluorophenyl)-5-methylisoxazol-4-yl-carbonylamino, nicotinylamino, benzoylamino, 4-bromobenzoylamino, 2,6-dimethoxybenzoylamino, formylamino, acetylamino, propionylamino, butyrylamino, isobutyrylamino, pivaloylamino, methoxycarbonylamino, benzyloxycarbonylamino, 1-amino-cyclohexylcarbonylamino, 2-amino-cyclohexylcarbonylamino, 3-ethoxynaphthoylamino, 2-(2-amino-4-thiazolyl)-2-ethylidene-acetylamino, 2-(2-amino-4-thiazolyl)-2-chloromethylene-acetylamino, phthalimido, succinimido, 1,2-cyclohexanedicarboxyimido, 2-(trimethylsilyl)ethoxycarbonylamino, 2,2-dimethyl-5-oxo-4-phenyl-imidazolidino, 4-(carbamoylcarboxymethylene)-1,3-dithiethane-2-yl-carbonylamino, etc.

Specific examples of the acylamino group represented by the formula

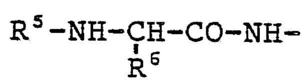

include, for example, D-alanyl amino, benzyl-Nα-carbobenzoxy-γ-D-glutamyl-D-alanylamino, D-phenylglycyl-D-alanylamino, N-carbobenzoxy-D-alanylamino, N-carbobebzoxy-D-phenylglycylamino, D-alanyl-D-phenylglycylamino, γ-D-glutamyl-D-alanylamino, 2-(4-ethyl-2,3-dioxo-1-piperazinocarboxamido)-2-phenylacetylamino, 2-(4-cyclohexyl-2,3-dioxo-1-piperazinocarboxamido)-2-phenylacetylamino, 2-(4-ethyl-2,3-dioxo-1-piperazinocarboxamido)-2-(4-sulfoxyphenyl)acetylamino, N-(4-ethyl-2,3-dioxo-1-piperazinocarboxamido)-D-alanylamino, N-(4-ethyl-2,3-dioxo-1-piperazinocarboxamido)-D-phenylglycylamino, 2-(2-amino-4-thiazolyl)-2-(4-ethyl-2,3-dioxo-1-piperazinocarboxamido)acetylamino, 2-(4-hydroxy-6-methylnicotinamido)-2-phenylacetylamino, 2-(4-hydroxy-6-methylnicotinamido)-2-(4-hydroxyphenyl)acetylamino, 2-{5,8-dihydro-2-(4-formyl-1-piperazinyl)-5-oxopyrido[2,3-d]pyrimidine-6-carboxamido}-2-phenylacetylamino, 2-(3,5-dioxo-1,2,4-triazine-6-carboxamido)-2-(4-hydroxyphenyl)acetylamino, 2-(3-furfurylideneamino-2-oxoimidazolidine-1-carboxamido)-2-phenylacetylamino, 2-(coumarin-3-carboxamido)-2-phenylacetylamino, 2-(4-hydroxy-7-methyl-1,8-naphthylidene-3-carboxamido)-2-phenylacetylamino, 2-(4-hydroxy-7-trifluoromethylquinolin-3-carboxamido)-2-phenylacetylamino, N-[2-(2-amino-4-thiazolyl)acetyl]-D-phenylglycylamino, 2-(6-bromo-1-ethyl-1,4-dihydro-4-oxothieno[2,3-b]pyridine-3-carboxamido)-2-phenylacetylamino, 2-(4-ethyl-2,3-dioxo-1-

piperazinocarboxamido)-2-thienylacetylamino, 2-(4-n-pentyl-2,3-dioxo-1-piperazinocarboxamido)-2-thienylacetylamino, 2-(4-n-octyl-2,3-dioxo-1-piperazinocarboxamido)-2-thienylacetylamino, 2-(4-cyclohexyl-2,3-dioxo-1-piperazinocarboxamido)-2-thienylacetylamino, 2-[4-(2-phenylethyl)-2,3-dioxo-1-piperazinocarboxamido]-2-thienylacetylamino, 2-(3-methylsulfonyl-2-oxoimidazolidine-1-carboxamido)-2-phenylacetylamino, 2-(3-furfurylideneamino-2-oxoimidazolidine-1-carboxamido)-2-(4-hydroxyphenyl)-acetylamino, 2-(4-ethyl-2,3-dioxo-1-piperazinocarboxamido)-2-(4-benzyloxyphenyl)acetylamino, 2-(4-ethyl-2,3-dioxo-1-piperazinocarboxamido)-2-(4-methoxyphenyl)acetylamino, 2-(8-hydroxy-1,5-naphthylidine-7-carboxamido)-2-phenylacetylamino, 2-(2-amino-4-thiazolyl)-2-formamidoacetylamino, 2-(2-amino-4-thiazolyl)-2-acetoamidoacetylamino, 2-phenyl-2-ureidoacetylamino, 2-phenyl-2-sulfo-ureidoamino, 2-thienyl-2-ureidoacetylamino, 2-amino-3-sulfamoylpropionylamino, 2-amino-2-(1H-indol-3-yl)acetylamino, 2-amino-2-(3-benzo[b]thienyl)acetylamino, 2-amino-2-(2-naphthyl)acetylamino, D-phenylglycyl, D-2-amino-(4-hydroxyphenyl)acetylamino, D-2-amino-2-(1,4-cyclohexadienyl)acetylamino, D-2-amino-2-(1-cyclohexenyl)-acetylamino, D-2-amino-2-(3-chloro-4-hydroxyphenyl)acetylamino, 2-hydroxymethylamino-2-phenylacetylamino, 2-(1-cyclohexenyl)-2-(4-ethyl-2,3-dioxo-1-piperazinocarboxamido)acetylamino, N-[2-(4-ethyl-2,3-dioxo-1-piperazinocarboxamido]-D-threonylamino, 2-guanylcarboxamido-2-phenylacetylamino, 2-(4-ethyl-2,3-dioxo-1-piperazinocarboxamido)-2-(3,4-dihydroxyphenyl)acetylamino, 2-(4-carboxy-5-imidazolylcarboxamido)-2-phenylacetylamino, 2-amino-2-(3-methylsulfonamidophenyl)acetylamino, etc.

Specific examples of the acylamino group represented by the formula $R^8$-$R^9$-CO-NH-include, for example, N-[2-(2-amino-4-thiazolyl)-2-methoxyiminoacetyl]-D-alanylamino, N-[2-(2-amino-4-thiazolyl)-2-methoxyiminoacetyl]-D-phenylglycylamino, 2-(2-amino-4-thiazolyl)-2-[2-(2-amino-4-thiazolyl)-2-methoxyiminoacetamido]acetylamino, 2-(2-chloroacetamido-4-thiazolyl)-2-methoxyiminoacetylamino, 2-(2-amino-4-thiazolyl)-2-methoxyiminoacetylamino, 2-(2-amino-4-thiazolyl)-2-ethoxyiminoacetylamino, 2-(2-amino-4-thiazolyl)-2-isopropoxyiminoacetylamino, 2-(2-amino-4-thiazolyl)-2-butoxyiminoacetylamino, 2-(2-amino-4-thiazolyl)-2-cyclopropylmethyloxyiminoacetylamino, 2-(2-amino-4-thiazolyl)-2-benzyloxyiminoacetylamino, 2-(2-amino-4-thiazolyl)-2-allyloxyiminoacetylamino, 2-(2-amino-4-thiazolyl)-2-[(1-methyl-1-carboxyethyl)oxyimino]acetylamino, 2-(2-amino-4-thiazolyl)-2-[(1-methyl-1-methoxycarbonylethyl)-oxyimino]acetylamino, 2-(2-amino-4-thiazolyl)-2-carboxymethyloxyiminoacetylamino, 2-(2-amino-4-thiazolyl)-2-carboxyvinyloxyiminoacetylamino, 2-(2-amino-4-thiazolyl)-2-carboxyethyloxyiminoacetylamino, 2-(2-amino-4-thiazolyl)-2-methoxycarbonylethyl oxyiminoacetylamino, 2-(2-amino-5-chloro-4-thiazolyl)-2-methoxyiminoacetylamino, 2-(2-amino-5-bromo-4-thiazolyl)-2-methoxyiminoacetylamino, 2-(2-amino-4-thiazolyl)-2-oxyiminoacetylamino, 2-thienyl-2-methoxyiminoacetylamino, 2-furyl-2-methoxyiminoacetylamino, 2-(1,2,4-thiadiazol-3-yl)-2-methoxyiminoacetylamino, 2-(1,2,4-thiadiazol-5-yl)-2-methoxyiminoacetylamino, 2-(1,3,4-thiadiazolyl)-2-methoxyiminoacetylamino, 2-(4-hydroxyphenyl)-2-methoxyiminoacetylamino, 2-phenyl-2-methoxyiminoacetylamino, 2-phenyl-2-oxyiminoacetylamino, 2-[4-(γ-D-glutamyloxy)phenyl]-2-oxyiminoacetylamino, 2-[4-(3-amino-3-carboxypropoxy)phenyl]-2-oxyiminoacetylamino, 2-thienyl-2-oxyiminoacetylamino, 2-(5-amino-1,2,4-thiadiazol-3-yl)-2-methoxyiminoacetylamino, 2-(5-amino-1,2,4-thiadiazol-3-yl)-2-ethoxyiminoacetylamino, 2-(5-amino-1,2,4-thiadiazol-3-yl)-2-carboxymethyloxyiminoacetylamino, 2-(5-amino-1,2,4-thiadiazol-3-yl)-2-[(1-methyl-1-carboxyethyl)oxyimino]acetylamino, 2-(2-amino-4-thiazolyl)-2-(2-amino-2-carboxy)ethyloxyiminoacetylamino, 2-(2-amino-4-thiazolyl)-2-(dimethylamidomethyloxyimino)acetylamino, 2-(2-amino-4-thiazolyl)-2-(3,4-diacetoxy-benzoyloxyimino)-acetylamino, 2-(2-amino-4-thiazolyl)-2-(1-carboxy-cyclopropyloxyimino)acetylamino, 2-(2-amino-4-thiazolyl)-2-(1-carboxycyclobutyloxyimino)acetylamino, 2-(2-amino-4-thiazolyl)-2-(2-imidazolylmethyloxyimino)-acetylamino, 2-(2-amino-4-thiazolyl)-2-(2-methyl-4-nitro-1-imidazolylethyloxyimino)acetylamino, 2-(2-amino-4-thiazolyl)-2-(3-pyrazolylmethyloxyimino)acetylamino, 2-(2-amino-4-thiazolyl)-2-(1H-tetrazol-5-yl-methyloxyimino)acetylamino, 2-(2-amino-4-thiazolyl)-2-(2-oxo-3-pyrrolidinyloxyimino)acetylamino, 2-[2-(2-amino-2-carboxyethylthio)]-4-thiazolyl-2-methoxyiminoacetylamino, . 2-(2-thioxo-4-thiazolidinyl)-2-methoxyiminoacetyl amino, etc.

Specific examples of the acylamino group represented by the formula $R^{15}$

$$\underset{\displaystyle R^{16}}{\underset{|}{-\text{CH}}}\text{-CO-NH-}$$

include, for example, 2-phenyl-2-sulfoacetylamino, 2-hydroxy-2-phenylacetylamino, 2-phenyl-2-sulfamoylacetylamino, 2-carboxy-2-phenylacetylamino, 2-(4-hydroxyphenyl)-2-carboxyacetylamino, 2-phenoxycarbonyl-2-phenylacetylamino, 2-phenyl-2-tolyloxycarbonylacetylamino, 2-(5-indanyloxycarbonyl)-2-phenylacetylamino, 2-formyloxy-2-phenylacetylamino, 2-alanyloxy-2-phenylacetylamino, 2-carboxy-2-thienylacetylamino, 2-(2-methylphenoxycarbonyl)-2-thienylacetylamino, 2-(2-amino-4-thiazolyl)-2-hydroxyacetylamino, 2-[4-(2-amino-2-carboxyethoxycarboxamido)phenyl]-2-hydroxyacetylamino, etc.

Specific examples of the acylamino group represented by the formula $R^{17}$-$R^{18}$-$CH_2$-CO-NH-include, for example, cyanoacetylamino, phenylacetylamino, phenoxyacetylamino, trifluoromethylthioacetylamino, cyanomethylthioacetylamino, difluoromethylthioacetylamino, 1H-tetrazolyl-1-acetylamino, thienylacetylamino, 2-(2-amino-4-thiazolyl)acetylamino, 4-pyridylthioacetylamino, 2-thienylthioacetylamino, 3,5-dichloro-1,4-dihydro-4-oxopyridine-1-acetylamino, β-carboxyvinylthioacetylamino, 2-(2-aminomethylphenyl)acetylamino, 2-chloroacetylamino, 3-aminopropionylamino, (2-amino-2-carboxy)ethylthioacetylamino, 4-amino-3-hydroxybutyrylamino, 2-carboxyethylthioacetylamino, 2-benzyloxycarbonylamino-acetylamino, β-carbamoyl-β-fluorovinylthioacetylamino, 2-(1-isopropylamino-1-isopropyliminomethylthio)acetylamino, 2-[1-(2-dimethylaminoethyl)-1H-tetrazol-5-yl-thio]acetylamino, 2-(1-methyl-1,3,5-triazol-2-yl)acetylamino, 2-(4-cyano-3-hydroxy-5-isothiazolylthio)acetylamino, etc.

Specific examples of the group represented by the formula

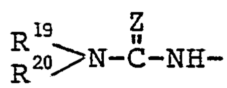

include, for example, carbamoylamino, methylaminocarbonylamino, ethylaminocarbonylamino, t-butylaminocarbonylamino, isobutylaminocarbonylamino, dimethylaminocarbonylamino, 2-methylphenylaminocarbonylamino, phenylaminocarbonylamino, 3-chlorophenylaminocarbonylamino, 4-nitrophenylaminocarbonylamino, 4-bromophenylaminocarbonylamino, thiocarbamoylamino, methylaminothiocarbonylamino, ethylaminothiocarbonylamino, phenylaminothiocarbonylamino, dimethylaminocarbonylamino, 3-fluorophenylaminocarbonylamino, etc.

Specific examples of the group represented by the formula $R^{21}$-NH-include, for example, methylamino, ethylamino, allylamino, cyclohexylamino, cyclohexylmethylamino, 4-chlorobenzylamino, phenylamino, 2-imidazolylamino, 1-methyl-2-imidazolylamino, 2-(2-amino-4-thiazolyl)-2-methoxyimino-thioacetylamino, 1-benzyl-4-pyridiniumamino, 2-acetyl-1-methylvinylamino, etc.

Specific examples of the alkylamino group represented by the formula

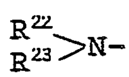

include, for example, dimethylamino, diethylamino, dipropylamino, dibenzylamino, dicyclohexylamino, N-benzyl-N-methylamino, diallylamino, N-phenyl-N-methylamino, pyrrolidinyl, piperidinyl, piperazinyl, morpholinyl, etc.

Specific examples of the alkylamino group represented by the formula

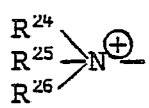

include, for example, trimethylammonium, triethylammonium, tribenzylammonium, benzyldimethylammonium, methylpyrrolidinium, methylpiperidinium, etc.

Specific examples of the alkenylamino group represented by the formula

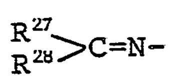

include, for example, dimethylaminomethyleneamino, 1-dimethylaminoethylideneamino, hexahydro-1H-azepine-1-ylmethyleneamino, 1-(N-benzyl-N-methylamino)ethylideneamino, 4-dimethylaminobenzylideneamino, (p-nitro)benzylidene-amino, benzylideneamino, etc.

Specific examples of the thioamino group represented by the formula $R^{29}$-$SO_n$ -NH-include, for example, benzenesulfonylamino, 4-methylbenzenesulfonylamino, 4-methoxybenzenesulfonylamino, 2,4,6-trimethylbenzenesulfonylamino, benzylsulfonylamino, 4-methylbenzylsulfonylamino, trifluoromethylsulfonylamino, phenacylsulfonylamino, methylsulfonylamino, ethylsulfonylamino, 4-fluorobenzenesulfonylamino, benzenesulfinylamino, 2-nitrobenzenesulfinylamino, 2,4-dimethylbenzenesulfinylamino, 4-chlorobenzenesulfinylamino, 4-methoxybenzenesulfinylamino, phenylthioamino, 2,4-dinitrophenylthioamino, triphenylmethylthioamino, 2-nitro-4-methoxyphenylthioamino, etc.

Specific examples of the silylamino group represented by the formula

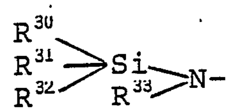

include, for example, trimethylsilylamino, triethylsilylamino, t-butyldimethylsilylamino, t-butyldiphenylsilylamino, isopropyldimethylsilylamino, triphenylsilylamino, triisopropylsilylamino, tribenzylsilylamino, (triphenylmethyl)dimethylsilylamino, 2,2,5,5-tetramethyl-2,5-disilylazacyclopentane, etc.

Specific examples of the formula

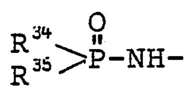

include, for example, dimethylphosphoamino, diethylphosphoamino, diphenylphosphoamino, dibenzylphosphoamino, di-4-chlorophenylphosphoamino, etc.

Specific examples of the group represented by the formula $R^{36}$-CO-CO-NH-include, for example, methoxalylamino, ethoxalylamino, phenoxalylamino, benzyloxalylamino, pyruvoylamino, ethyloxalylamino, oxamoylamino, benzylaminooxalylamino, thienyloxalylamino, 2-amino-4-thiazolyl-oxalylamino, ethylaminooxalylamino, etc.

Specific examples of the group represented by the formula -$COOR^{37}$ include, for example, methylester, ethylester, n-propylester, isopropylester, t-butylester, t-amylester, benzylester, 4-bromobenzylester, 4-nitrobenzylester, 2-nitrobenzylester, 3,5-dinitrobenzylester, 4-methoxybenzylester, benzhydrylester, phenacylester, 4-bromo-phenacylester, phenylester, 4-nitrophenylster, methoxymethylester, methoxyethoxymethylester, ethoxymethylester, benzyloxymethylester, acetoxymethylester, pivaloyloxymethylester, 2-methylsulfonylethylester, 2-trimethylsilylethylester, methylthiomethylester, tritylester, 2,2,2-trichloroethylester, 2-iodoethylester, cyclohexylester, cyclopentylester, allylester, cinnamylester, 4-picolylester, 2-tetrahydropyranylester, 2-tetrahydrofuranylester, trimethylsilylester, t-butyldimethylsilylester, t-butyldiphenylsilylester, acetylmethylester, 4-nitrobenzoylmethylester, 4-mesylbenzoylmethylester, phthalimidomethylester, propionyloxymethylester, 1,1-dimethylpropylester, 3-methyl-3-butenylester, succinimidomethylester, 3,5-di-t-butyl-4-hydroxybenzylester, mesylmethylester, benzenesulfonyl methylester, phenylthiomethylester, iminomethylaminoethylester, 1-iminoethylaminoethylester, dimethylaminoethylester, pyridine-1-oxido-2-methylester, methylsulfinylmethylester, bis-(4-methoxyphenyl)methylester, 2-cyano-1,1-dimethylethylester, t-butyloxy-carbonylmethylester, benzoylaminomethylester, 1-acetoxyethylester, 1-isobutyryloxyethylester, 1-ethoxycarbonyloxyethylester, phthalide ester, 4-t-butylbenzylester, 5-indanylester, 5-methyl-2-oxo-1,3-dioxolen-4-yl-methylester, 5-t-butyl-2-oxo-1,3-dioxolen-4-yl-methylester, etc.

Specific examples of the group represented by the formula

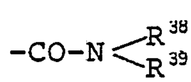

include, for example, dimethylamido, diethylamido, dipropylamido, dibenzylamido, dicyclohexylamido, N-benzyl-N-methylamido, N-phenyl-N-methylamido, pyrrolidineamido, piperidineamido, piperazineamido, morpholineamido, carboxymethylamido, 1-carboxyethylamido, etc.

The compound (I) of the present invention can be prepared by a <u>per se</u> conventional reaction of a combination of <u>per se</u> conventional reactions, employing as a starting material, for example, 2-(4-formylamido-3-oxo-2-isoxazolidinyl)-5-oxo-2-tetrahydrofurancarboxylic acid benzhydrylester (III) [cf. the specification of EP Publication 0,191,989 A1.

The reaction route is schematically shown, for example, as follows

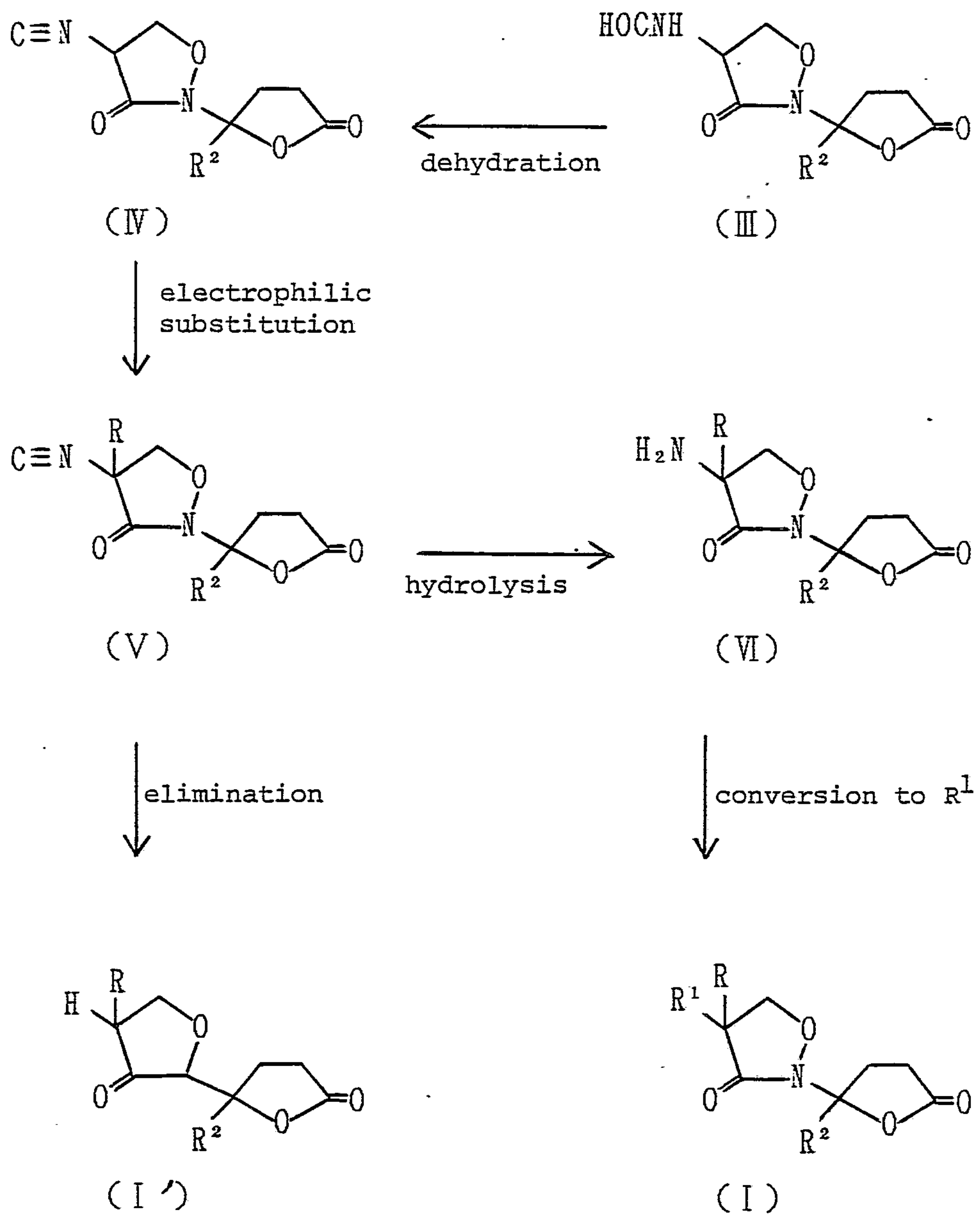


[in the formulae, each symbol is of the same meaning as defined above]

By subjecting (I), (I′) or (V) obtained as above to a per se conventional reaction, a compound (I) having substituents corresponding to $R^1$, R and $R^2$ can be prepared.

And, by removing the group represented by R, a compound in which a double bond is formed between R and the adjacent carbon atom can be prepared.

The above-mentioned per se conventional reactions will be explained as follows.

Synthesis of isocyano compound (III)

Dehydration reaction of 4-formamide derivative (II) is conducted by, for example, allowing trichloromethyl chloroformate to act on the compound (II) in an anhydrous non-polar solvent in the presence of a tertiary amine. As the solvent, use is made of dichloromethane, chloroform, dichloroethane, dioxane, tetrahydrofuran, acetonitrile, etc., singly or in a suitable combination thereof. As the tertiary amine, use is made of trimethylamine, triethylamine, tri-n-butylamine, N,N-dimethyl cyclohexylamine, N,N-dimethyl aniline, pyridine, quinoline, etc. This reaction is conducted by dissolving or suspending the starting compound in a solvent as mentioned above, to which are added tertiary amine then trichloromethyl chloroformate to thereby allow the reaction to undergo. Use of 2.1 mol. of tertiary amine and 0.5 mol. of trichloromethyl chloroformate relative to 1 mol. of the 4-formamide derivative is preferable. The reaction proceeds at a temperature range of from -60°C to 0°C. For preventing hydrolysis of the reaction product, the resulting reaction solution is preferably neutralized with a cold saturated aqueous solution of sodium hydrogencarbonate after completion of the reaction.

Synthesis of (V) bv electrophilic substitution reaction

Electrophilic substitution reaction on the compound (IV) is conducted by first processing (IV) with a base in an organic solvent to cause formation of carbanion at α-position of the isonitrile group, on which various electrophilic reagents are allowed to act. As the solvent, use is made of tetra hydrofuran, dimethyl sulfoxide, dichloromethane, benzene, dimethylformamide, methanol, ethanol, etc. As the base, use is made of n-butyl lithium, sodium hydroxide, sodium cyanide, lithium diisopropylamide, potassium carbonate, triethylamine, tri-n-butylamine, 1,8-diazabicyclo[5,4,0]undec-7-ene (DBU), etc. As the electrophilic reagent, use is made of relatively highly reactive substituted lower alkyl halide such as benzyl bromide, phenacyl bromide, bromoacetic acid ester, etc., acrylic acid ester, methyl methanethiosulfonate. methoxycarbonyl disulfide, and lower oxo-alkane such as acetone, aldehyde, etc. This reaction is preferably conducted by dissolving or suspending a compound (IV) in a solvent mentioned as above, then by adding thereto 0.5 - 2 mol. of a base and 1.0 - 1.5 mol. of an electrophilic reagent to allow the reaction to proceed. The reaction temperature is in the range from -60°C to 30°C, and the reaction time is within the range of from 0.5 - 3 hours. The substituent R of thus obtained compound (V) can be further converted to another substituent. For example, methylthio group can be converted to a functional group such as methoxy, formamido, azido, etc. by processes similar to per se known ones, respectively. And, when R is hydroxymethyl, a number of derivatives through oxygen by the reaction with a variety of nucleophilic reagents. Among them, an active ester such as a sulfonic acid derivative can be converted to a variety of substituted methyl derivatives by the reaction of a nucleophilic reagent. As such substituted methyl derivatives are mentioned acyloxy methyl, carbamoyloxy methyl, methanesulfonyloxy methyl, permethyl of bromine, chlorine, iodine, etc., cyanomethyl, azidomethyl, aminomethyl, acylaminomethyl, etc.

Synthesis of (I') by elimination reaction of isocyano group :

Compound (I') can be prepared by subjecting a corresponding compound (V) to the reaction of eliminating the isocyano group. This elimination reaction can be conducted in the presence of a reducing agent such as di(or tri)-alkyltin hydride e.g. tri(n-tert. butyl)tin hydride, triaryl tin e.g. triphenyltin hydride. The reaction is conducted in a non-protonic solvent such as tetrahydrofuran, dimethylformamide, dioxane, benzene, toluene, etc. in the presence of a catalytic amount of azobisisobutyronitrile. The reaction temperature is not specifically limitative, but the reaction is conducted under cooling or under heating, and the reaction time ranges from 30 minutes to 3 hours. When the R of compound (I') obtained by this reaction is a hydroxymethyl derivative, it can be converted to an exomethylene derivative by further subjecting to dehydration.

Hyrolysis of (V):

Compound (VI) can be prepared by subjecting a corresponding compound (V) to hydrolysis by applying the method utilized in the field of penicillin. This reaction is conducted usually in an organic solvent by allowing p-toluenesulfonic acid monohydrate to act on the reaction system. As the organic solvent, use is often made of acetone, dichloromethane, tetrahydrofuran, dimethylformamide, etc. The reaction temperature is not specifically limitative, but preferably ranges from 0°C to 50°C, and the reaction time is within the range of from 15 minutes to 60 minutes. The compound (VI) is obtained as p-toluene sulfonic acid sometimes in crystalline form, but usually, without isolation, further subjected in situ to a reaction for forming an organic residue through nitrogen.

Reaction for forming organic residue through nitrogen of compound (VI) :

The reaction, which involves reacting the compound (VI) with a compound capable of introducing a group forming the organic residue bonded through nitrogen,includes, for example, acylation, ureidization (thioureidization), alkylation, alkenylation, thionation, silylation, phosphorylation reactions, etc.

Acylation

Acylation of the amino group can be carried out by reacting a starting compound with an acylating agent containing the acyl group in the group $R^1$, such as a reactive derivative of carboxylic acid, in a solvent. As the reactive of carboxylic acid, there are used, for example, acid halides, acid anhydrides, amide compounds, active thioesters, etc., and specific examples of such a reactive derivative are mentioned in the following.

1) Acid halides :

As the acid halide as employed herein, there are used, for example, acid chlorides, or acid bromides.

2) Acid anhydrides :

As the acid anhydrides as employed herein, there are used, for example, mono alkyl carbonic acid mixed acid anhydrides, mixed acid anhydrides comprising aliphatic carboxylic acids (e.g., acetic acid, pivalic acid, valeric acid, isovaleric acid, trichloroacetic acid, etc.), mixed acjd anhydrides comprising aromatic carboxylic acids (e.g. benzoic acid, etc.) or symmetric type acid anhdyrides.

3) Amide compounds :

As the amide compound as employed herein, there are used, for example, compounds having an acyl group attached to the nitrogen in the ring, such as pyrazole, imidazole, 4-substituted imidazole, dimethylpyrazole, benzotriazole, etc.

4) Active esters :

As the active ester as employed herein, there are used, for example, esters such as methyl esters, ethyl esters, methoxymethylesters, propargyl esters, 4-nitrophenyl esters, 2,4-dinitrophenylesters, trichlorophenyl esters, pentachlorophenylesters,-mesylphenyl esters, etc. as well as esters formed with 1-hydroxy-1H-2-pyridone, N-hydroxysuccinimide, N-hydroxyphthalimide, etc.

5) Active thioesters :

As the active thioester as employed herein, there are used. for example, thioesters formed with heterocyclic thiols such as 2-pyridylthiol, 2-benzothiazolylthiol, etc.

Various kinds of reactive derivatives as described above are selected depending upon the type of carboxylic acids.

This reaction is, in some instances, carried out in the presence of a base as exemplified by aliphatic tertiary amines (e.g., trimethylamine, triethylamine, tripropylamine, tri-n-butylamine, etc.), tertiary amines such as N-methylpiperidine, N-methylpyrrolidine, cyclohexyl dimethylamine, N-methylmorpholine,etc., dialkylamines such as di-n-butylamine, diisobutylamine, dicyclohexylamine, etc., aromatic amines such as pyridine, lutidine, $\gamma$-collidine etc., hydroxides or carbonates of alkali metals such as lithium, sodium, potassium, etc. , hydroxides or carbonates of alkaline earth metals such as alkaline earth metals e.g., calcium, magnesium, etc.

In this procedure, the reactive derivative of carboxylic acid is normally used at a ratio of about 1 mole per mole of the compound (VI), but it can be used in excess as well, unless it affects adversely the reaction. In the case of a base being used, the amount of such base to be used is normally about 1 to 30 moles per mole of the compound (VI), preferably about 1 to 10 moles, varying with the types of the starting compound (VI) used and reactive derivatives of carboxylic acid employed and other reaction conditions. This reaction is carried out normally in a solvent. As the said solvent, there are used conventional solvents, either alone or as a mixture, for example, ethers such as dioxane, tetrahydrofuran, diethyl ether, diisopropyl ether, propylene oxide, butylene oxide, etc., esters such as ethyl acetate, ethyl formate, etc., halogenated hydrocarbons such as chloroform, dichloromethane, 1,2-dichloroethane, 1,1,1-trichloroethane, etc., hydrocarbons such as benzene, toluene, n-hexane, etc., amides such as N,N-dimethylformamide, N,N-dimethylacetamide, etc., nitriles such as acetonitrile, etc., etc. In the above mentioned bases, the liquid ones can also be used to allow them to serve dual purposes of the base and solvent. The reaction temperature is not specifically limited, as far as the reaction proceeds, but the reaction is conducted normally at about -50°C to 150°C, preferably about -30°C to 80°C. The reaction goes to termination normally within several ten minutes to several ten hours, varying with the types of starting compounds, bases, reaction temperatures and kinds of the solvent then employed, but it requires, in some instances, several ten days.

Ureido formation (thioureido formation)

The reaction of converting the amino group into the ureido or thioureido group is carried out by reacting the starting compound with a substituted isocyanate or substituted isothiocyanate containing a group represented by the abovedescribed formula

$$\begin{matrix} R^{19} \\ R^{20} \end{matrix} > N-C(=Z)-$$

(wherein $R^{19}$ , $R^{20}$ and Z are as defined in the foregoing) in the presence of a solvent. As the said substituted isocyanate, there are used, for example, methyl isocyanate, ethyl isocyanate, phenyl isocyanate, p-bromophenyl isocyanate, etc., while as the substituted isothiocyanate, there are employed, for example, methyl isothiocyanate, phenylisothiocyanate, etc. In this reaction, the substituted isocyanate or substituted isothiocyanate is normally used at a ratio of about 1 mole per mole of the compound (VI), but can also be employed in excess, unless it affects adversely the reaction. As the suitable solvent, there are sued, for example, tetrahydrofuran, diethyl ether, ethyl acetate, chloroform, dichloromethane, toluene, or the like. The reaction temperature is in the range of about -20°C to 50°C, and the reaction time ranges normally from about 10 minutes to 5 hours.

Alkylation

The reaction of combining the amino group of the compound (III) with a group bonded through carbon is to be described below as alkylation.

The alkylated derivative of the compound (VI) can be prepared by reacting the compound (VI) with an alkylating agent containing a group bonded to the relevant nitrogen of the group $R^1$ through carbon. As the alkylating agent, there are used, for example, halogenated alkyl compounds such as propyl chloride, butyl chloride, benzyl chloride, butyl bromide, benzyl bromide, allyl bromide, methyl iodide, ethyl iodide, propyl iodide, etc. dilakyl sulfate compounds such as dimethyl sulfate, diethyl sulfate, etc., substituted sulfonate compounds such as methyl mesylate, ethyl mesylate, methyl tosylate, ethyl tosylate, etc., dihalogenated alkyl compounds (e.g. 1,5-dichloropentane, 1,4-dichlorobutane,etc.), etc. This reaction is normally carried out in a solvent as exemplified by water, methanol, ethanol, benzyl alcohol, benzene, dimethylformamide, tetrahydrofuran, acetonitrile, etc. The temperature of this reaction is about 20°C to 200°C, while the reaction time ranges from about 30 minutes to 50 hours. This reaction, by changing the reaction conditions such as a molar ratio of the compound (VI) to the alkylating agent, permits selective production of a secondary amine, tertiary amine or quaternary amine compound. It is also possible to introduce different substituent groups into the nitrogen, by conducting the reaction stepwise. The reaction of introducing a group bonded through carbon other than alkyl groups can be carried out by procedurcres similar to the above.

Alternatively, the said alkylation can also be conducted by combining a compound (VI) with a carbonyl compound in the presence of a reducing agent. Examples of the reducing agent employable in this reaction include lithium aluminium hydride, sodium cyanoborohydride, sodium borohydride, sodium, sodium amalgam and combinations of zinc with an acid. Also, the reaction can be carried out by catalytic reducting using for example palladium, platinum, rhodium, etc. as a catalyst. The reaction of converting the amino group into a compound represented by $R^{21}$ -NH-(imino-substituted alkylamino, alkylimino-substituted alkylamino or substituted guanidino group):

The reaction of converting the amino group into an iminosubstituted alkylamino or alkylimino-substituted alkylamino group is carried out reacting the starting compound with, for example, imidoesters in a solvent such as dioxane, tetrahydrofuran, dimethylformamide, chloroform, acetone, acetonitrile, water, etc. Suitable imidoesters to be employed include, for example, methyl formimidate, ethyl formimidate, benzyl formimidate, methyl acetoimidate, ethyl acetoimidate, methylphenyl acetoimidate, ethyl N-methylformimidate, methy N-ethylformimidate, methyl N-isopropylformimidate, etc. The reaction temperature is in the neighbourhood of 0°C to 25°C, and the reaction time ranges normally from 1 to 6 hours. The reaction of converting the amino group into guanidino group is conducted by reacting the starting compound with, for example, O-alkyl or O-aryl pseudourea or S-alkyl or S-alkyl-or S-aryl pseudothioureas in a solvent such as water, dimethylformamide, hexamethylenephosphoramide, etc. As the above pseudoureas, there are used, for example, O-methyl pseudoureas, O-2,4-dichlorophenyl pseudourea, O-N,N-trimethyl pseudourea, etc. and as the above pseudothioureas, there are employed, for example, S-p-nitrophenyl pseudothiourea, etc. The reaction temperature is in the neighbourhood of 0°C to 40°C, while the reaction time is normally in the range of 1 to 24 hours.

Alkenylation (imination) :

Alkenylation (imination) of the compound (VI) can be carried out by dehydrative condensation of the compound (VI) with a carbonyl compound. This reaction proceeds in the absence of a solvent, but can also be conducted in a solvent. An acid or a base is, in some instances, used as a catalyst. The objective compound can also be prepared by heating under reflux the compound (VI) and a carbonyl compound in the presence of a dehydrating agent or with use of a dehydration apparatus such as Dean-Stark. The solvent, which is usable in this reaction, includes, for example, benzene, toluene, dichloromethane, ethanol, etc. The reaction temperature ranges from about 1 hour to 20 hours. The acid, which is used as a catalyst, includes, for example, benzenesulfonic acid, methanesulfonic acid, sulfuric acid, boron trifluoride, zinc chloride, etc., while the base includes, for example, potassium hydroxide, sodium carbonate, etc. The dehydrating agent, which is useful in this reaction, includes, for example, molecular sieves, silica gel, anhydrous magnesium sulfate, anhydrous sodium sulfate, etc.

Thionation :

The thionation reaction for the compound (VI) is normally carried out by allowing the compound (VI) to react with a halogenated thio compound (e.g. halogenated sulfonyl, halogenated sulfinyl, halogenated sulfenyl) containing a group represented by the formula $R^{29}$ -SOn-(wherein $R^{29}$ and n are defined as above) in a solvent in the presence of a base. The solvent, which is used in this reaction, includes, for example, water, acetone, dioxane, dimethylformamide, benzene, tetrahydrofuran, dichloromethane, or a suitable mixture thereof. As the base, there are used, for example, organic bases, such as pyridine, picoline, triethylamine, diisopropyl ethylamine, N-methylmorpholine, etc. or inorganic bases, such as sodium hydroxide, potassium hydroxide, sodium carbonate, potassium carbonate, etc. In this reaction, about one equivalent of a halogenated thio compound and about 1 to 10 equivalents of a base against the compound (VI) are normally used. The reaction temperature ranges from about -20°C to 80°C, and the reaciton time is within the range of from 15 minutes to 10 hours.

This reaction is also conducted using a thioacid anhydride (e.g. toluenesulfonic anhydride, trifluoromethanesulfonic anhydride, etc.) in place of the halogenated thio compound. Also, this reaction can be carried out by allowing the starting compound to react with a thionating reagent such as N-sulfonyl-N-methylpyrrolidinium, N-sulfonylimidazolide, N-sulfonyl-1H-1,2,4-triazolide, etc.

Silylation :

The silylation reaction for the compound (VI) can be carried out normally by allowing the compound (VI) to react with a halogenated silyl compound (e.g. silyl chloride compounds, silyl bromide compounds, etc.) containing a group represented by the formula

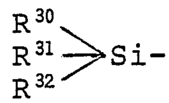

or $R^{33}$-(wherein $R^{30-33}$ are as defined hereinbefore) in the presence of a base. The base includes, for example, organic bases such as pyridine, picoline, triethylamine, diisoprooyl ethylamine, N-methylmorpholine, etc. The reaction is preferably carried out in a solvent, as exemplified by acetone, dioxane, dimethylformamide, benzene, tetrahydrofuran, dichloromethane, etc. The reaction temperature is about -20°C to 80°C, while the reaction time ranges from about 15 minutes to 20 hours.

Phosphorylation :

The phosphorylation reaction for the compound (VI) is normally carried out by allowing the compound (VI) to react with an approximately equimolar amount of a phosphoryl chloride (e.g. dimethylphosphoryl chloride, diethylphosphoryl chloride, diphenylphosphoryl chloride, dibenzylphosphoryl chloride, etc.) containing a group represented by the formula

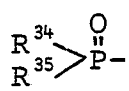

(wherein $R^{34}$ and $R^{35}$ are as defined hereinbefore) in a solvent in the presence of an approximately equimolar or excessive amount of a base. As the base, use is made of organic bases such as pyridine, picolins, triethylamine, N-methylmorpholine, etc. or inorganic bases such as sodium hydroxide, potassium hydroxide, sodium hydrogencarbonate, sodium carbonate, etc. As the solvent, use is made of, for example, water, acetone, acetonitrile, dioxane, dimethylformamide, tetrahydrofuran, dichlormethane, etc., either alone or as a suitable mixture. The reaction temperature is about -20°C to 80°C, and the reaction time ranges from 15 minutes to 15 hours.

The reaction of the compound (I) with a compound capable. of introducing a group bonded to the carbon or oxygen atom of the carboxy, which is derivable from carboxy, is carried out by, for example, subjecting to esterification or amidation.

Esterification of carboxylic acid :

The esterification is carried out by, for example, the following procedures :

1) The compound (I, wherein $R^2$ stands for carboxy, the same applies hereinafter) is allowed to react with a diazoalkane such as diazomethane, phenyldiazomethane, diphenyldiazomethane, etc. in a solvent such as tetrahydrofuran, dioxane, ethyl acetate, acetonitrile, etc. at about 0°C to its refluxing temperature for about 2 minutes to 2 hours.

2) An alkali metal salt of the compound (I) is allowed to react with an activated alkyl halide such as methyl iodide, benzyl bromide, p-nitro-benzyl bromide, m-phenoxybenzyl bromide, p-t-butylbenzyl bromide, pivaloyloxymethyl chloride, etc. Referring to the suitable reaction conditions, the reaction is allowed to proceed in a solvent such as dimethylformamide, dimethylacetamide or hexamethylenephosphoramide at about 0°C to 60°C for about 2 minutes to 4 hours. Coexistence of triethylamine etc. in this reaction solution does not affect adversely the proceeding of reaction.

3) The compound (I) is allowed to react with an alcohol such as methanol, ethanol, benzyl alcohol, etc. This reaction is carried out in the presence of a carbodiimide condensing agent such as dicyclohexylcarbodiimide, etc. at about 0°C to the refluxing temperature of the solvent then employed for about 15 minutes to 18 hours. As the solvent, use is made of chloroform, dichloromethane, dichloroethane, etc.

4) An acid anhydride of the compound (I) formed by allowing the compound (I) to react with an acid chloride, for example, ethyl chloroformate, benzyl chloroformate, etc. is allowed to react with an alcohol such as those mentioned in the above 3) under the reaction conditions described in the above 3). This acid anhydride is obtainable by allowing the compound (I) to react with the acid chloride in a solvent such as tetrahydrofuran, dichloromethane, etc. at 25°C to the refluxing temperature for about 15 minutes to 10 hours.

5) The compound (I) is allowed to react with a silylating agent such as trimethylsilyl chloride, t-butyldimethylsilyl chloride in the copresence of triethylamine, etc. in a solvent e.g. dichloromethane, chloroform, tetrahydrofuran, etc. at about 0°C to the refluxing temperature for about 15 minutes to 16 hours.

Amidation of carboxylic acid :

The amidation of carboxylic acid is carried out by synthesizing an acid anhydride of the compound (I) from the compound (I) and an acid anhydride such as trimethylacetyl chloride, ethyl chloroformate, benzyl chloroformate or an acid anhydride e.g. acetic anhydride, trifluoroacetic anhydride, etc., followed by allowing thus-synthesized acid anhydride to react with ammonia or a selected amine e.g. the above-mentioned alkyl-, dialkyl-aralkyl-or heterocyclic amine reagent.

The above reaction is carried out in a solvent such as dichloromethane, tetrahydrofuran, dimethylformamide, etc. at about 0°C to the refluxing temperature for 15 minutes to 16 hours.

Deprotection reaction :

When the objective compound (I) thus obtained has a protective group at the carboxyl group, the protective group can be removed upon necessity. For removing the said protective group, a suitable procedure can be selected from conventional ones such as the procedure using an acid, the procedure using a base, the procedure using hydrazine or the procedure by means of reduction, depending on the type of such protective groups. In the case of resorting to the procedure using an acid, the acid employable includes, for example, inorganic acids such as hydrochloric acid, sulfuric acid, phosphoric acid, etc., organic acids such as formic acid, acetic acid, trifluoroacetic acid, propionic acid, etc., as well as acidic ion exchange resins, etc., while varying with the type of such protective groups and other conditions. In the case of resorting to the procedure using a base, the base employable includes, for example, inorganic bases such as hydroxides and carbonates of alkali metals (e.g. sodium, potassium etc.) and alkaline earth metals (e.g. calcium, magnesium, etc., organic bases such as metal alkoxides, organic amine, quaternary ammonium salts, as well as basic ion exchange resins, etc., while varying with the type of such protective groups and other conditions. When a solvent is used in the case of the above-described procedure employing an acid or a base, hydrophilic organic solvents, water or a mixture solvent are often used.

In the case of the procedure by means of reduction, a procedure of using a metal such as tin, zinc, etc. or a metal compound such as chromium dichloride, chromium acetate, etc. and an organic or inorganic acid such as acetic acid, propionic acid, hydrochloric acid, a procedure comprising reduction in the presence of a metal catalyst for catalytic reduction, while varying with the type of such protective groups and other conditions. The catalyst employable for the catalytic reduction is exemplified by a platinum catalyst such as platinum line, platinum sponge, platinum black, platinum oxide, colloidal platinum, etc., a palladium catalyst such as palladium sponge, palladium black, palladium oxide, palladium barium sulfate, palladium barium carbonate, palladium carbon, palladium-silica gel, colloidal palladium, etc., reduced nickel, nickel oxide, Raney nickel, Urushibara nickel, etc. In the case of the procedure comprising reduction by using a metal and an acid, a metal such as iron, chromium, etc. and an inorganic acid such as hydrochloric acid, etc. or an organic acid such as formic acid, acetic acid, propionic acid, etc. The procedure by means of reduction is normally carried out in a solvent exemplified most commonly by alcohols such as methanol, ethanol, propyl alcohol, isopropyl alcohol, etc., ethyl acetate, etc. In the procedure comprising the use of a metal and an acid, water, acetone, etc. are often employed, and the acid is in the state of liquid, the acid itself can be used as a solvent.

In the cases of a procedure using an acid, a procedure using a base and a procedure comprising reduction, the reaction temperature is normally within the range from that under cooling to that under warming.

The compound (VI), which is a compound of the above formula (I) wherein $R^1$ is amino, can be produced by allowing a compound of the formula (I) wherein $R^1$ is an organic residue bonded through nitrogen other than amino to undergo a reaction similar to the above-mentioned deprotection reaction.

The compound (VI) is useful as a starting compound (intermediate for synthesis) for the production of the compound (I) wherein R is an organic residue bonded through nitrogen other than amino.

Further, the compound (I) wherein $R^2$ is carboxyl can be produced by allowing a compound (I) wherein $R^2$ is a group derivable from carboxyl group to undergo a reaction similar to the above-described deprotection reaction.

The objective compound (I) thus obtained can be isolated and purified by a per se known means such as concentration, conversion of liquid nature, phasic transfer, solvent extraction, lyophilization, crystallization, recrystallization, fractional distillation, chromatography, etc.

The presence of two asymmetric carbons in the basic skeleton allows theoretically the objective compound (I) to exist in four kinds of stereoisomers, and their individual stereoisomers and mixtures thereof fall into the scope of this invention. Similarly, occurrence of any asymmetric carbon in the groups represented by $R^1$ and $R^2$ results in existence of stereoisomers, and their individual stereoisomers and mixtures thereof are also included in the scope of this invention. In cases where the above-described reaction affords these stereoisomers as a mixture, their individual stereoisomers can be isolated by a conventional method such as various chromatographic procedures, recrystallization, etc.

The compound (I) of this invention can, in some instances, act on a base to give a salt. The base includes, among others, inorganic bases such as sodium, potassium, lithium, magnesium, ammonia, etc. and organic bases such as pyridine, collidine, triethylamine, triethanolamine, etc.

The compound (I) of this invention, when obtained in the free form, may be allowed to form a salt by resorting to a conventional means, and the compound (I) obtained as a salt may be converted to the free form by a conventional means.

Also, the compound (I) forms, in some instances, the intramolecular salt, which falls within the scope of this invention as well.

The stereoisomers of the compound (I), either alone or as a mixture, can be used as medicines.

The compound (I) thus obtained is useful as a medicine, having, for example, antimicrobial activity against certain species of gram-positive and gram-negative bacteria.

## Examples

### Example 1

Production of diphenylmethyl 2-[(4S)-4-isocyano-3-oxo-2-ioxazolidinyl]-5-oxo-2-tetrahydrofuran carboxylate :

In 400 mℓ of dry methylene chloride was dissolved 5.2 g of diphenyl methyl 2-[(4S)-4-formamido-3-oxo-2-isoxazolidinyl]-5-oxo-2-tetrahydrofuran carboxylate. To the solution were added, under cooling with acetone - dry ice, 4 g of pyridine and 0.8 mℓ of trichloromethyl chloroformate (diphosgene), and the mixture was stirred for one hour at the same temperature. The reaction solution was shaken with a saturated aqueous solution of sodium hdyrogen carbonate. Then, the methylene chloride layer was dried over anhydrous magnesium sulfate, which was concentrated under reduced pressure. The concentrate was subjected to a silica-gel column chromatography, eluting with ethyl acetate - n-hexane (1:1) to give 3.5 g of the above-titled compound as orange-colored powder. IR $\nu_{max}^{KBr}$ $cm^{-1}$:

2150,1800,1765,1740,1490,1450,1295,1260,1170,1060,740,700

NMR($CDCl_3$,ppm): 2.10~3.50(4H,m),4.00~4.90(3H,m),7.00(1H,s),7.35(10H,s)

Example 2

Production of diphenylmethyl 2-[(4RS)-4-isocyano-4-methylthio-3-oxo-2-isoxazolidinyl]-5-oxo-2-tetrahydrofuran carboxylate :

In 5 mℓ of anhydrous dimethylformamide was dissolved 1.0 g of diphenylmethyl 2-[(4S )-4-isocyano-3-oxo-2-isoxazolidinyl]-5-oxo-2-tetrahydrofuran carboxylate. To the solution was added 0.4 g of methyl methanethiosulfonate. The mixture was cooled, to which was added 0.37 g of anhydrous potassium carbonate. The mixture was stirred at the same temperature for one hour. The reaction solution was poured into water, which was subjected to extraction with ethyl acetate. The extract was washed with water and dried over anhydrous magnesium sulfate. The resultant was concentrated under reduced pressure, and the concentrate was subjected to a silica-gel column chromatography, eluting with ethyl acetate - n-hexane (1:2) to give 0.6 g of the above-titled compound. IR $\nu_{max}^{KBr}$ $cm^{-1}$:

2120,1800,1765,1730,1490,1450,1170,1060,695
NMR($CDCl_3$,ppm): 2.10~3.50(4H,m),2.33 (3H,s),4.10~4.75(2H,m),7.00(1H,s),7.20~7.50(10H,br.s)

Example 3

Production of diphenylmethyl 2-[(4RS)-4-isocyano-4-hydroxymethyl-3-oxo-2-isoxazolidinyl]-5-oxo-2-tetrahydrofuran carboxylate :

In 3 mℓ of anhydrous dimethylformamide was dissolved 203 mg of diphenylmethyl 2-[(4S)-4-isocyano-3-oxo-2-isoxazolidinyl]-5-oxo-2-tetrahydrofuran carboxylate. To the solution were added, under cooling, 0.5 mℓ of a 37% aqueous solution of formalin and 70 mg of pulverized potassium carbonate, and the mixture was stirred for 40 minutes at the same temperature. The reaction solution was poured into water, which was subjected to extraction with ethyl acetate. The extract was washed with water, dried over anhydrous magnesium sulfate and concentrated under reduced pressure. The concentrate was subjected to a silica-gel column chromatogrpahy, eluting ethyl acetate - n-hexane (1:1) to give 142 mg of the above-titled compound. IR $\nu_{max}^{KBr}$ $cm^{-1}$:

2140,1800,1765,1740,1180,1060,695
NMR($CDCl_3$,ppm): 2.10~3.60(5H,m),3.80~4.05(2H,m),4.10~4.80(2H,m),7.00(1H,s),7.20~7.50(10H,br.s)

Example 4

Production of diphenylmethyl 2-[(4RS)-4-isocyano-4-(2-hydroxy-2-propyl)-3-oxo-2-isoxazolidinyl]-5-oxo-2-tetrahydrofuran carboxylate :

In 8 mℓ of acetone was suspended 812 mg of diphenylmethyl 2-[(4S)-4-isocyano-3-oxo-2-isoxazolidinyl]-5-oxo-2-tetrahydrofuran carboxylate. To the suspension was added 276 mg of pulverized potassium carbonate, and the mixture was stirred for 2.5 hours under cooling with cold water. The reaction solution was concentrated under reduced pressure. The concentrate was subjected to extraction with ethyl acetate. The extract was washed with water, dried over anhydrous magnesium sulfate and concentrated under reduced pressure. The concentrate was subjected to a silica-gel column chromatography, eluting with ethyl acetate - n-hexane (1:1) to give 510 mg of the above-titled compound. IR $\nu_{max}^{Nujol}$ $cm^{-1}$:

2130,1795,1760,1725,1175,1060,725,700
NMR($CDCl_3$,ppm): 1.40(6H,s),2.20~3.45(5H,m),4.00~4.80(2H,m),7.00(1H,s),7.20~7.50(10H,br.s)

Example 5

Production of diphenylmethyl 2-[(4RS)-4-isocyano-4-(diphenylmethyloxycarbonylmethyl)-3-oxo-2-isoxazolidinyl]-5-oxo-2-tetrahydrofuran carboxylate :

In 3 mℓ of anhydrous dimethylformamide was dissolved 800 mg of diphenylmethyl 2-[(4S)-4-isocyano-3-oxo-2-isoxa zolidinyl]-5-oxo-2-tetrahydrofuran carboxylate. To the solution were added, under cooling, 660 mg of diphenylmethyl bromoacetate and 272 mg of pulverized potassium carbonate. The mixture was stirred for 3 hours at the same temperature. The reaction solution was poured into water, which was subjected to extraction with ethyl acetate. The ethyl acetate layer was washed with water dried over anhydrous magnesium sulfate and concentrated under reduced pressure. The concentrate was subjected to a silica-gel column chromatography, eluting with ethyl acetate - n-hexane (1:2), to give 750 mg of the above-titled compound. IR $\nu_{max}^{KBr}$ $cm^{-1}$:

2130,1805,1765,1730,1495,1450,1375,1060,740,690
NMR($CDCl_3$,ppm): 2.20~3.50(6H,m),4.50(2H,s),6.93(1H,s),7.00(1H,s),7.20~7.50(20H,br.s)

Example 6

Production of diphenylmethyl 2-[(4RS)-4-acetoxymethyl-4-isocyano-3-oxo-2-isoxazolidinyl]-5-oxo-2-tetrahydrofuran carboxylate :

In 8 mℓ of methylene chloride was dissolved 436 mg of diphenylmethyl 2-[(4RS)-4-isocyano-4-hydroxymethyl-3-oxo-2-isoxazolidinyl]-oxo-2-tetrahydrofuran carboxylate. To the solution were added, under cooling, 118 mg of pyridine and 153 mg of acetic anhydride. The mixture was then stirred for 3 hours at room temperatures. The reaction solution was washed with water, dried over anhydrous sodium sulfate and concentrated under reduced pressure. The concentrate was eluted with ethyl acetate - n-hexane (1:2) to give 382 mg of the above-titled compound. IR $\nu_{max}^{KBr}$ $cm^{-1}$:

2140,1805,1760,1730(s),1495,1450,1220,1180,1060,700
NMR($CDCl_3$,ppm): 2.03(3H,s),2.20~3.45(4H,m),4.10~4.60(2H,m),4.40(2H,s),6.30(1H,s),7.15~7.45(10H,br.s)

Example 7

Production of diphenylmethyl 2-[(4RS)-4-chloroacetylcarbamoyloxymethyl-4-isocyano-3-oxo-2-isoxazolidinyl]-5-oxo-tetrahydrofuran carboxylate :

In 20 mℓ of dry methylene chloride was suspended 970 mg of diphenylmethyl 2-[(4RS)-4-hydroxymethyl-4-isocyano-3-oxo-2-isoxasolidinyl]-5-oxo-2-tetrahydrofuran carboxylate. To the suspension was added, under ice-cooling, 400 mg of chloroacetyl isocyanate. The mixture was stirred for one hour at the same temperature, followed by concentration under reduced pressure. The concentrate was subjected to a silica-gel chromatography, eluting with ethyl acetate -n-hexane (1:1), to give 520 mg of the above-titled compound. IR $\nu_{max}^{KBr}$ $cm^{-1}$:

2130,1800,1765,1490,1190,1050,750,690
NMR($CDCl_3$,ppm): 2.10~3.60(4H,m),4.25~4.80(6H,m),7.00(1H,s),7.20~7.50(10H,m),8.40~8.60(1H,br.s)

Example 8

Production of sodium 2-{(4RS)-4-[2-(2-amino-4-thiazolyl)-(Z)-2-(methoxyimino)acetamido]-4-methylthio-3-oxo-2-isoxazolidinyl}-5-oxo-2-tetrahydrofuran carboxylate [Compound (8c)]:

(a) Production of diphenylmethyl 2-{(4RS)-4-[2-(2-chloroacetamido-4-thiazolyl)-(Z)-2-(methoxyimino)-acetamido]-4-methylthio-3-oxo-2-isoxazolidinyl}-5-oxo-2-tetrahydrofuran carboxylate [Compound (8a)] :

In 20 mℓ of methylene chloride was dissolved 1.52 g of diphenylmethyl 2-[(4RS)-4-isocyano-4-methylthio-3-oxo-2-isoxazolidinyl]-5-oxo-2-tetrahydrofuran carboxylate. To the solution was added, under ice-cooling, 0.8 g of p-toluenesulfonic acid monohydrate. The mixture was stirred at the same temperature for 2.5 hours, to which was then added an aqueous solution of sodium hydrogen carbonate to render the reaction mixture alkaline. The methylene chloride layer was dried over anhydrous magnesium sulfate, followed by concentration under reduced pressure. To the concentrate were added 10 mℓ of dimethylacetamide and 0.9 g of pyridine. To the mixture was added, under ice-cooling, 1.3 g of 2-(2-chloroacetamido-4-thiazolyl)-(Z)-2-methoxyiminoacetic acid chloride hydrochloride, followed by stirring at the same temperature for 40 minutes. The reaction mixture was poured into water, which was subjected to extraction with ethyl acetate. The organic layer was washed with water, dried over anhydrous magnesium sulfate and concentrated under reduced pressure. The concentrate was subjected to a silica-gel column chromatography, eluting with ethyl acetate : n-hexane (2:1) to give 0.7 g of the Compound (8a) as a pale yellow foamy product. IR $\nu_{max}^{KBr}$ $cm^{-1}$:

1810,1770,1690,1545,1270,1180,1050,740,700

NMR($CDCl_3$,ppm): 2.10(3H,S),2.20~3.40(4H,m),4.05(3H,s),4.20(2H,s),4.40~5.0(2H,m),7.00(1H,s),7.20~7.50-(10H,m)

(b) Production of diphenylmethyl 2-{(4RS)-4-[2-(2-amino-4-thiazolyl)-(Z)-2-(methoxyimino)acetamido -4-methylthio-3-oxo-2-isoxazolidinyl}-5-oxo-2-tetrahydrofuran carboxylate [Compound (8b)] :

In 4 mℓ of dimethylformamide was dissolved 425 mg of the Compound (8a) obtained as above. To the solution was added, under ice-cooling, 106 mg of sodium N-methyldithiocarbamate. The mixture was stirred at the same temperature for 2.5 hours, which was poured into water, followed by extraciton with ethyl acetate. The extract solution was washed with water, dried over anhydrous magnesium sulfate, then concentrated under reduced pressure. The concentrate was subjected to a silica-gel column chromatography, eluting with ethyl acetate : n-hexane (2:1), to give 233 mg of the Compound (8b). IR $\nu_{max}^{KBr}$ $cm^{-1}$:

1805,1760,1730(s),1675,1610,1530,1180,1050,750,695

NMR(DMSO-$d_6$,ppm): 2.20(3H,s),2.20~3.40(4H,m),4.00(3H,s),4.40~4.90(2H,m),6.05~6.25(2H,br.s),7.00(1H,s)-,7.10~7.50(10H,br.s),8.75,8.85(1H,br.s)

(c) Production of the above-titled Compound (8c) :

To 185 mg of the Compound (8b) was added 2 mℓ of 99% formic acid, and the mixture was stirred at room temperature for 1.5 hour. Formic acid was distilled off under reduced pressure. The residue was neutralized by the addition of an aqueous solution of sodium hydrogen carbonate, which was subjected to a CHP-20 column chromatography. The portion eluted with a 10% aqueous solution of ethanol was freeze-dried to give 109 mg of the above-titled Compound (8c) as colorless powder. IR $\nu_{max}^{KBr}$ $cm^{-1}$:

1770,1715,1655,1525,1370,1190,1030,

NMR(DMSO-$d_6$,ppm): 2.15(3H,s),2.10~3.20(4H,m),3.80(3H,s),4.35~4.70(2H,m),6.70(1H,s),7.00~7.30(2H,br.s)-,9.45~9.55(1H,br.s)

Example 9

Production of sodium 2-{(4RS)-4-[2-(2-amino-4-thiazolyl)-(Z)-2-(methoxyimino)acetamido]-4-hydroxymethyl-3-oxo-2-isoxazolidinyl}-5-oxo-2-tetrahydrofuran carboxylate [Compound (9c)] :

(a) Production of diphenylmethyl 2-{(4RS)-4-[2-(2-chloroacetamido-4-thiazolyl)-(Z)-2-(methoxyimino)-acetamido]-4-hydroxymethyl-3-oxo-2-isoxazolidinyl}-5-oxo-2-tetrahydrofuran carboxylate [Compound (9a)] :

In 3 mℓ of chloroform was suspended 436 mg of diphenylmethyl 2-[(4RS)-4-hydroxymethyl-4-isocyano-3-oxo-2-isoxazolidinyl]-5-oxo-2-tetrahydrofuran carboxylate. To the suspension was added, under ice-cooling, 209 mg of p-toluenesulfonic acid monohydrate, and the mixture was stirred at the same temperature for one hour. The reaction solution was made alkaline with an aqueous solution of sodium hydrogen carbonate. The chloroform layer was dried over anhydrous magnesium sulfate, followed by concentration under reduced pressure. The concentrate was dissolved in 3 mℓ of dimethylacetamide. To the solution was added, under ice-cooling, 174 mg of pyridine and subsequently 366 mg of 2-(2-chloroacetamido-4-thiazolyl)-(Z)-2-methoxyiminoacetic acid chloride hydrochloride. The reaction was then allowed to proceed at the same temperature for two hours. The reaction solution was poured into water, which was subjected to extraction with ethyl acetate. The ethyl acetate layer was washed with water, dried over anhydrous magnesium sulfate and concentrated under reduced pressure. The concentrate was subjected to a silica-gel column chromatography, eluting with ethyl acetate : n-hexane (3:1) to give 170 mg of the above-titled Compound (9a). IR $\nu^{KBr}_{max}$ $cm^{-1}$:

1810,1770,1740,1670,1540,1180,1050,700

NMR($CDCl_3$,ppm): 2.10~3.50(4H,m),4.03(3H,s),4.23(2H,s),4.50~5.00(4H,m),7.00(1H,s),7.20~7.50(10H,m)

b) Production of diphenylmethyl 2-{(4RS)-4-[2-(2-amino-4-thiazolyl)-(Z)-2-(methoxyimino)acetamido]-4-hydroxymethyl-3-oxo-2-isoxazolidinyl}-5-oxo-tetrahydrofuran carboxylate [Compound (9b)] :

In 3 mℓ of dimethylformamide was dissolved 170 mg of the Compound (9a) obtained as above. To the solution was added, under ice-cooling, 48 mg of sodium N-methyldithiocarbamate, and the mixture was stirred at room temperature for one hour. The reaction solution was poured into water, which was subjected to extraction with ethyl acetate. The ethyl acetate layer was washed with water, dried over anhydrous magnesium sulfate, then concentrated under reduced pressure. The concentrate was subjected to a silica-gel column chromatography, eluting with ethyl acetate - n-hexane (2:1) to give 115 mg of the above-titled Compound (9b). IR $\nu^{KBr}_{max}$ $cm^{-1}$:

1805,1760,1735,1670,1535,1180,1050,700

NMR($CDCl_3$,ppm): 2.10~3.50(4H,m),4.00(3H,s),4.50~5.00(4H,m),5.10~5.30(2H,br.s),6.86(1H,s),7.00(1H,s)-,7.20~7.50(10H,br.s)

(c) Production of the above-titled Compound (9c) :

In 4 mℓ of formic acid was dissolved, under ice-cooling, 115 mg of the Compound (9b) obtained as above. The solution was allowed to undergo reaction at room temperature for one hour, from which was distilled off formic acid. The residue was adjusted to pH 7 with an aqueous soltuion of sodium hydrogen carbonate, which was then subjected to a CHP-20 column chromatography, eluting with a 10% aqueous solution of ethanol. The eluate was freeze-dried to give 47 mg of the above-titled Compound (9c). IR $\nu^{KBr}_{max}$ $cm^{-1}$:

1780,1730,1655,1530,1375,1180,1040

NMR($D_2O$,ppm external standard): 2.20~3.50(4H,m),4.00(3H,s),7.03(1H,s)

Example 10

Production of sodium 2-{(4RS)-4-[2-(2-amino-4-thiazolyl)-(Z)-2-(methoxyimino)acetamido]-4-acetoxymethyl-3-oxo-2-isoxazolidinyl}-5-oxo-2-tetrahydrofuran carboxylate [Compound (10c)] :

(a) Production of diphenylmethyl 2-{(4RS)-4-[2-(2-chloroacetamido-4-thiazolyl)-(Z)-2-(methoxyimino)-acetamido]-4-acetoxymethyl-3-oxo-2-isoxazolidinyl}-5-oxo-2-tetrahydrofuran carboxylate [Compound (10a)]:

In 3 mℓ of chloroform was dissolved 400 mg of diphenylmethyl 2-[(4RS)-4-acetoxymethyl-4-isocyano-3-oxo-2-isoxazolidinyl]-5-oxo-2-tetrahydrofuran carboxylate. To the solution was added, under ice-cooling, 175 mg of p-toluenesulfonic acid monohydrate. The mixture was stirred at the same temperature for one hour. The reaction solution was made alkaline with sodium hydrogen carbonate. The chloroform layer was dried over anhydrous magnesium sulfate, which was concentrated under reduced pressure. The concentrate was dissolved in 2 mℓ of dimethylacetamide. To the solution were added, under ice-cooling, 146 mg of pyridine then 305 mg of 2-(2-chloroacetamido-4-thiazolyl)-(Z)-2-methoxyiminoacetic acid chloride•hydrochloride. The mixture was stirred at the same temperature for one hour. To the reaction solution was added water, which was subjected to extraction with ethyl acetate. The ethyl acetate layer was washed with water, then dried over anhydrous magnesium sulfate, followed by concentration under reduced pressure. The concentrate was subjected to a silica-gel column chromatography, eluting with ethyl acetate - n-hexane (2:1) to give 510 mg of the above titled Compound (10a). IR $\nu_{max}^{KBr}$ $cm^{-1}$:

1805,1755,1680,1545,1260,1230,1180,1050,780,755,695
NMR($CDCl_3$,ppm): 2.06(3H,s),2.10~3.50(4H,m),4.03(3H,s),4.20(2H,s),4.30~4.80(4H,m),7.00(1H,s),7.15~7.45-(10H,m)

(b) Production of diphenylmethyl 2-{(4RS)-4-[2-(2-amino-4-thiazolyl)-(Z)-2-(methoxyimino)acetamido]-4-acetoxymethyl-3-oxo-2-isoxazolidinyl}-5-oxo-2-tetrahydrofuran carboxylate [Compound (10b)] :

In 2 mℓ of dimethylformamide was dissolved 470 mg of the Compound (10a) obtained as above. To the solution was added, under ice-cooling, 108 mg of sodium N-methyldithiocarbamate. The mixture was stirred at room temperature for one hour. To the reaction solution was added ethyl acetate, and the mixture was washed with water, dried over anhydrous sodium sulfate, followed by concentration under reduced pressure. The concentrate was subjected to a silica-gel colum chromatography, eluting with ethyl acetate - n-hexane (3:1) to give 251 mg of the above titled Compound (10b). IR $\nu_{max}^{KBr}$ $cm^{-1}$:

1805,1750,1675,1525,1045,750,690
NMR($CDCl_3$,ppm): 2.03(3H,s),2.10~3.50(4H,m),4.00(3H,s),4.40~4.90(4H,m),5.10~5.35(2H,br.s),7.00(1H,s)-,7.20~7.50(10H,m)

(c) Production of the above-titled Compound (10c) :

To 200 mg of the Compound (10b) obtained as above was added, under ice-cooling, 2 mℓ of 99% formic acid. The reaction was allowed to proceed at room temperature for one hour. The reaction solution was concentrated under reduced pressure, and the pH of the concentrate was adjusted to 7 with an aqueous solution of sodium hydrogen carbonate, which was subjected to a CHP-20 column chromatography, eluting with a 10% aqueous solution of ethanol. The eluate was freeze-dried to give 107 mg of the above-titled Compound (10c) as colorless powder. IR $\nu_{max}^{KBr}$ $cm^{-1}$:

1680,1740,1660,1530,1375,1240,1200,1040
NMR($D_2O$,ppm external standard): 2.39(3H,s),2.40~3.60(4H,m),4.23(3H,s),7.26(1H,s)

Example 11

Production of sodium 2-{(4RS)-4-[2-(2-amino-4-thiazolyl)-(Z)-2-(methoxyimino)acetamido]-4-carbamoyloxymethyl-3-oxo-2-isoxazolidinyl}-5-oxo-2-tetrahydrofuran carboxylate [Compound (11c)] :

(a) Production of diphenylmethyl 2-{(4RS)-4-[2-(2-chloroacetamido-4-thiazolyl)-(Z)-2-(methoxyimino)-acetamido]-4-chloroacetylcarbamoyloxymethyl-3-oxo-2-isoxazolidinyl}-5-oxo-2-tetrahydrofuran carboxylate [Compound (11a)] :

In 4 mℓ of chloroform was dissolved 480 mg of diphenylmethyl 2-[(4RS)-4-chloroacetylcarbamoyloxymethyl-4-isocyano-3-oxo-2-isoxazolidinyl]-5-oxo-2-tetrahydrofuran carboxylate. To the solution was added, under ice-cooling, 181 mg of p-toluenesulfonic acid. The mixture was stirred at the same temperature for one hour. The reaction solution was made alkaline with an aqueous solution of sodium hydrogen carbonate. The chloroform layer was then dried over anhydrous magnesium sulfate, followed by concentration under reduced pressure. The concentrate was dissolved in 2 mℓ of dimethylacetamide. To the solution was added 164 mg of pyridine. To the mixture was added, under ice-cooling, 345 mg of 2-(2-chloroacetamido-4-thiazolyl)-(Z)-2-methoxyiminoacetic acid chloride hydrochloride. The mixture was stirred at the same temperature for one hour. To the reaction solution was added water, which was subjected to extraction with ethyl acetate. The ethyl acetate layer was washed with water, dried over anhydrous magnesium sulfate, then concentrated under reduced pressure. The concentrate was subjected to a silica-gel column chromatography, eluting with ethyl acetate to give 540 mg of the above-titled Compound (11a). IR $\nu_{max}^{KBr}$ $cm^{-1}$:

1800,1760,1730,1685,1540,1495,1260,1190,1055,780,755,690
NMR($CDCl_3$,ppm): 2.20~3.60(4H,m),4.03(3H,s),4.29(2H,s),4.39(2H,s),4.30~4.80(4H,m),7.03(1H,s),7.20~7.50-(10H,br.s)

(b) Production of diphenylmethyl 2-{(4RS)-4-[2-(2-amino-4-thiazolyl)-(Z)-2-(methoxyimino)acetamido]-4-carbamoyloxymethyl-3-oxo-2-isoxazolidinyl}-5-oxo-2-tetrahydrofuran carboxylate [Compound(11b)] :

In 2 mℓ of dimethylformamide was dissolved 520 mg of the Compound (11a) obtained as above. To the solution was added, under ice-cooling, 217 mg of sodium N-methyldithiocarbamate. The mixture was stirred at the same temperature for 5 hours, to which was added water, followed by extraction with ethyl acetate. The ethyl acetate layer was washed with water, dried over anhydrous magnesium sulfate and concentrated under reduced pressure. The concentrate was subjected to a silica-gel column chromatography, eluting with ethyl acetate, to give 540 mg of the Compound (11a) titled above. IR $\nu_{max}^{KBr}$ $cm^{-1}$:

1800,1730,1675 1610,1515,1725,1260,1175,1040,690
NMR($CDCl_3$,ppm): 2.10~3.40(4H,m),4.00(3H,s),4.30~4.80(4H,m),5.20~5.40(2H,br.s),7.00(1H,s),7.20~7.50-(10H,br.s)

(c) Production of the above-titled Compound (11c) ;

In 4 mℓ of formic acid was dissolved under ice-cooling 150 mg of the Compound (11b) obtained as above, which was allowed to undergo reaction at room temperature for 1.5 hour, followed by distilling off formic acid under reduced pressure. To the residue was added an aqueous solution of sodium hydrogen carbonate to adjust its pH at 7, which was subjected to a CHP-20 column chromatography, eluting with a 5% aqueous solution of ethanol. The eluate was freeze-dried to give 76 mg of the above-titled Compound (11c). IR $\nu_{max}^{KBr}$ $cm^{-1}$:

1780,1730,1660,1530,1380,1335,1200,1085,1040
NMR($D_2O$,ppm external standard): 2.50~3.50(4H,m),4.20(3H,s),4.66~4.86(2H,m),7.26(1H,s)

Example 12

Production of sodium 2-{(4RS)-4-[2-(2-amino-4-thiazolyl)-2-(methoxyimino)acetamido]-4-diphenylmethyloxycarbonylmethyl-3-oxo-2-isoxazolidinyl}-5-oxo-2-tetrahydrofuran carboxylate [Compound (12c)] :

(a) Production of diphenylmethyl 2-{(4RS)-4-[2-(2-chloroacetamido-4-thiazolyl)-(Z)-2-(methoxyimino)-acetamido]-4-diphenylmethyloxycarbonylmethyl-3-oxo-2-isoxazolidinyl}-5-oxo-2-tetrahydrofuran carboxylate [Compound (12a)] :

In 8 mℓ of chloroform was dissolved 700 mg of diphenylmethyl 2-[(4RS)-4-diphenylmethyloxycarbonylmethyl-4-isocyano-3-oxo-2-isoxazolidinyl]-5-oxo-2-tetrahydrofuran carboxylate. To the solution was added, under ice-cooling, 232 mg of p-toluenesulfonic acid monohydrate. The mixture was stirred at the same temperature for one hour. The reaction solution was made alkaline with sodium hydrogen carbonate. The chloroform layer was then dried over anhydrous magnesium sulfate, followed by concentration under reduced pressure. The concentrate was dissolved in 3 mℓ of dimethylacetamide. To the solution were added, under ice-cooling, 211 mg of pyridine and, subsequently, 443 mg of 2-(2-chloroacetamido-4-thiazolyl)-(Z)-2-methoxyiminoacetic acid chloride•hydrochloride. The mixture was stirred at the same temperature for one hour. To the reaction solution was added water, which was subjected to extraciton with ethyl acetate. The ethyl acetate layer was washed with water, dried over anhydrous magnesium sulfate and concentrated under reduced pressure. The concentrate was subjected to a silica-gel column chromatography, eluting with ethyl acetate - n-hexane (1:2), to give 458 mg of the above-titled Compound (12a). IR $\nu_{max}^{KBr}$ $cm^{-1}$:

1805,1760(s),1735,1680,1540,1175,1040,750,690
NMR($CDCl_3$,ppm): 2.10~3.40(6H,m),3.96(3H,s),4.23(2H,s),4.53(2H,s),6.86(1H,s),7.00(1H,s),7.20~7.50(20H,m)

(b) Production of diphenylmethyl 2-{(4RS)-4-[2-(2-amino-4-thiazolyl)-(Z)-2-(methoxyimino)acetamido]-4-diphenylmethyloxycarbonylmethyl-3-oxo-2-isoxazolidinyl}-5-oxo-2-tetrahydrofuran carboxylate [Compound (12b)] :

In 2 mℓ of dimethylformamide was dissolved 445 mg of the Compound (12a) obtained as above. To the solution was added, under ice-cooling, 85 mg of sodium N-methyldithiocarbamate. The mixture was stirred at room temperature for two hours. To the reaction solution was added water, which was subjected to extraction with ethyl acetate. The extract was washed with water and dried over anhydrous magnesium sulfate, which was concentrated under reduced pressure. The concentrate was subjected to a silica-gel column chromatography, eluting with ethyl acetate - n-hexane (2:1) to give 389 mg of the above-titled Compound (12b). IR $\nu_{max}^{KBr}$ $cm^{-1}$:

1805,1760(s),1730,1675,1605,1525,1170,1040,745,690
NMR($CDCl_3$,ppm): 2.10~3.50(6H,m),3.93(3H,s),4.50(2H,s),5.05~5.15(2H,br.s),6.90(1H,s),7.00(1H,s),7.20~7.50-(20H,m)

(c) Production of the above-titled Compound (12c) :

In 4 mℓ of formic acid was dissolved, under ice-cooling, 310 mg of the Compound (12b) obtained as above. The solution was stirred at room temperature for 1.5 hour, from which was distilled off formic acid under reduced pressure. The pH of the residue was adjusted to 7 with an aqueous solution of sodium hydrogen carbonate. The resultant was subjected to a CHP-20 column chromatography, eluting with a 50% aqueous solution of ethanol. The eluate was freeze-dried to give 85 mg of the above-titled Compound (12c). IR $\nu_{max}^{KBr}$ $cm^{-1}$:

1775,1730,1660,1525,1370,1190,1035,690
NMR($D_2O$,ppm): 6.90(1H,s)7.20~7.50(10H,m),2.50~3.50(4H,m),4.10(3H,s)

Example 13

Production of diphenylmethyl 2-{(4RS)-4-azido-4-[2-(2-chloroacetamido-4-thiazolyl)-(Z)-2-(methoxyimino)-actamido]-3-oxo-2-isoxazolidinyl}-5-oxo-2-tetrahydrofuran carboxylate :

In 5 mℓ of chloroform was dissolved 616 mg of diphenylmethyl 2-[(4RS)-4-isocyano-4-methylthio-3-oxo-2-isoxazolidinyl]-5-oxo-2-tetrahydrofuran carboxylate. To the solution was added, under ice-cooling, 250 mg of p-toluenesulfonic acid monohydrate, and the mixture was stirred at the same temperature for one hour. The reaction solution was made alkaline with sodium hydrogen carbonate. The chloroform layer was dried over anhydrous magnesium sulfate, which was concentrated under reduced pressure. The concentrate was dissolved in 2 mℓ of dimethylacetamide. To the solution were added, under cie-cooling, 0.2 mℓ of pyridine and 5 mℓ of a pyridinium azide - methylene chloride solution of 1.3 m mole concentration. The mixture was then cooled to -20°C, to which was added 271 mg of mercuric chloride, followed by stirring for 30 minutes. The resultant was subjected to filtration. The filtrate was was washed with water and dried over anhydrous magnesium sulfate, which was concentrated under reduced pressure. The oily concentrate was dissolved in 2 mℓ of dimethylacetamide. To the solution were added, under ice-cooling, 200 mg of pyridine and, subsequently, 500 mg of 2-(2-chloroacetamido-4-thiazolyl)-(Z)-2-methoxyiminoacetic acid chloride hydrochloride, followed by stirring at room temperature for 40 minutes. The reaction solution was poured into water, which was subjected to extraction with ethyl acetate. The extract was washed with water, dried over anhydrous magnesium sulfate and concentrated under reduced pressure. The concentrate was subjected to a silica-gel column chromatography, eluting with ethyl acetate - n-hexane (1:1) to give 40 mg of the above-titled compound. IR $\nu_{max}^{KBr}$ $cm^{-1}$:

2120,1810,1770,1735,1690,1540,1260,1180,1060,1040,690
NMR($CDCl_3$,pp): 2.10~3.40(4H,m),4.00(3H,s),4.20(2H,s),4.46,4,86(2H,d,d,J=9Hz),7.00(1H,s),7.10~7.40-(10H,m),7.76~7.96(10H,br.s)

Example 14

Production of diphenylmethyl 2-(4RS)-4-hydroxymethyl-3-oxo-2-isoxazolidinyl]-5-oxo-2-tetrahydrofuran carboxylate :

In 20 mℓ of dry benzene was suspended 878 mg of diphenylmethyl 2-[(4RS)-4-hydroxymethyl-4-isocyano-3-oxo-2-isoxazolidinyl]-5-oxo-2-tetrahydrofuran carboxylate. To the suspension were added 0.6 mℓ of tri-n-butyltin hydride and azobisisobutyronitrile of a catalytic amount. The mixture was subjected to heating under reflux in argon stream for 30 minutes. The solvent was distilled off under reduced pressure. The residue was washed several times with n-hexane by decantation. The remaining insoluble oily substance was then subjected to a silica-gel column chromatography, eluting with ethyl acetate - n-hexane (1:1) to give 560 mg of the above-titled compound. IR $\nu_{max}^{Nujol}$ $cm^{-1}$:

1785,1760,1615,1260,1180,1050
NMR($CDCl_3$,ppm): 2.15~3.50(5H,m),3.70~4.60(4H,m),6.96(1H,s),7.20~7.50(10H,m)

Example 15

Production of diphenylmethyl 2-[(4RS)-4-methanesulfonyloxymethyl-3-oxo-2-isoxazolidinyl]-5-oxo-2-tetrahydrofuran carboxylate :

In 2.4 mℓ of pyridine was dissolved 1.2 g of diphenylmethyl 2-[(4RS)-4-hydroxymethyl-3-oxo-2-isoxazolidinyl]-5-oxo-2-tetrahydrofuran carboxylate. To the solution was added, under ice-cooling, 0.24 mℓ of methanesulfonyl chloride. The mixture was stirred at the same temperature for 30 minutes, which was then poured into ice-water, followed by extraction with ethyl acetate. The organic layer was washed with dilute hydrochloric acid and a saturated aqueous saline solution in sequence, followed by drying over anhydrous sodium sulfate. The solvent was distilled off under reduced pressure to give 1.4 g of the above-titled compound. IR $\nu_{max}^{Nujol}$ $cm^{-1}$:

1785,1760,1720,1175,1060,700
NMR($CDCl_3$,ppm): 2.10~3.50(5H,m),3.63(3H,s),4.10~4.70(4H,m),6.96(1H,s),7.20~7.50(10H,m)

Example 16

Production of diphenylmethyl 2-[(4RS)-4-azidomethyl-3-oxo-2-isoxazolidinyl]-5-oxo-2-tetrahydrofuran carboxylate :

In 10 mℓ of dimethylsulfoxide was dissolved 1.4 g of diphenylmethyl 2-[(4RS)-4-methanesulfonyloxymethyl-3-oxo-2-isoxazolidinyl]-5-oxo-2-tetrahydrofuran carboxylate. To the solution were added, under ice-cooling, 0.45 g of sodium iodide and 0.39 g of sodium azide. The mixture was stirred at room temperature for 30 minutes. The reaction solution was poured into ice-water, which was subjected to extraction with ethyl acetate. The organic layer was washed with water, which was then dried over anhydrous sodium sulfate, followed by distilling off the solvent under reduced pressure. The residue was subjected to a silica-gel column chromatography, eluting with ethyl acetate - n-hexane (1:2) to give 0.49 g of the above-titled compound. IR $\nu_{max}^{Nujol}$ $cm^{-1}$:

2100,1790,1760,1720,1170,1060
NMR($CDCl_3$,ppm): 2.10~3.45(5H,m),3.45~4.70(4H,m),6.98(1H,s),7.20~7.50(10H,m)

Example 17

Production of sodium 2-{(4RS)-4-[2-(2-amino-4-thiazolyl)-(Z)-2-(methoxyimino)acetamidomethyl]-3-oxo-2-isoxazolidinyl}-5-oxo-2-tetrahydrofuran carboxylate [Compound (17b)] :

(a) Production of sodium 2-{(4RS)-4-[2-(2-chloroacetamido-4-thiazolyl)-(Z)-2-(methoxyimino)-acetamidomethyl]-3-oxo-2-isoxyzolidinyl}-5-oxo-2-tetrahydrofuran carboxylate [Compound (17a)] :

In 4 mℓ of a 50% tetrahydrofuran - water was dissolved 218 mg of diphenylmethyl 2-[(4RS)-4-azidomethyl-3-oxo-2-isoxazolidinyl]-5-oxo-2-tetrahydrofuran carboxylate. To the solution was added 220 mg of 5% palladium carbon. The mixture was stirred under ice-cooling for 30 minutes in hydrogen stream. The catalyst was then filtered off. To the filtrate was added 2 mℓ of tetrahydrofuran. To the mixture were added, under ice-cooling, 168 mg of sodium hydrogen carbonate and 150 mg of 2-(2-chloroacetamido-4-thiazolyl)-(Z)-2-methoxyiminoacetic acid chloride hydrochloride. The mixture was stirred at the same temperature for one hour. The reaction solution was shaken with ethyl acetate. The aqueous layer was separated and subjected to a CHP-20 column chromatography, eluting with 30% ethanol -water. The eluate was freeze-dried to give 105 mg of the above-titled compound (17a) as colorless powder. IR $\nu_{max}^{KBr}$ $cm^{-1}$:

1770,1700(s),1650,1540,1370,1190
NMR($D_2O$,ppm extarnal standard): 2.35~5.00(9H,m),4.03(3H,s),4.45(2H,s),7.50(1H,s)

(b) Production of the above-titled Compound (17b) :

In 2 mℓ of 50% tetrahydrofuran - water was dissolved 105 mg of the Compound (17a) obtained as above. To the solution was added, under ice-cooling, 65 mg of sodium N-methyldithiocarbamate, followed by stirring at room temperature for one hour. The reaction solution was shaken with ethyl acetate, and the aqueous layer was separated, which was subjected to a CHP-20 column chromatography, eluting with water. The eluate was freeze-dried to give 40 mg of the above-titled Compound (17b) as pale yellow powder. IR $\nu_{max}^{KBr}$ $cm^{-1}$:

1780,1710,1660,1530,1380,1190,1040
NMR($D_2O$,ppm external standard): 2.35~5.00(9H,m),4.00(3H,s),6.97(1H,s)

Example 18

Production of diphenylmethyl 2-[(4RS)-4-methylthio-3-oxo-2-isoxazolidinyl]-5-oxo-2-tetrahydrofuran carboxylate :

In 10 mℓ of dry benzene was dissolved 452 mg of diphenylmethyl 2-[(4RS)-4-isocyano-4-methylthio-3-oxo-2-isoxazolidinyl]-5-oxo-2-tetrahydrofuran carboxylate. To the solution were added 0.3 mℓ of tri-n-butyltin hydride and a catalytic amount of azobisisobutyronitrile. The mixture was heated under reflux for one hour under argon stream . The solvent was distilled off under reduced pressure. The residue was subjected to a silica-gel column chromatography, eluting with acetonitrile - methylene chloride (0.5 : 9.5) to give 235 mg of the above-titled compound. IR $\nu_{max}^{Nujol}$ $cm^{-1}$:

1790,1760,1705,1270,1180,1060
NMR($CDCl_3$,ppm): 2.20(3H,s),2.20~3.50(4H,m),3.50~3.90(1H,m),3.95~4.80(2H,m),7.00(1H,s),7.25~7.50-(10H,m)

Example 19

Production of sodium 2-[(4RS)-4-methylthio-3-oxo-2-isoxazolidinyl]-5-oxo-2-tetrahydrofuran carboxylate :

To 3 mℓ of 99% formic acid was added 150 mg of diphenylmethyl 2-[(4RS)-4-methylthio-3-oxo-2-isoxazolidinyl]-5-oxo-2-tetrahydrofuran carboxylate, and the mixture was stirred at room temperature for one hour, which was concentrated under reduced pressure. The concentrate was washed with n-hexane. To insolubles were added 2 mℓ of water and 80 mg of sodium hydrogen carbonate to neutralize, followed by shaking with ethyl acetate. The aqueous layer was subjected to a CHP-20 column chromatogrpahy, eluting with 5% ethanol -water. The eluate was freeze-dried to give 65 mg of the above-titled compound. IR $\nu_{max}^{KBr}$ $cm^{-1}$:

1775,1710,1645,1380,1200,1115
NMR($D_2O$,ppm external standard): 2.25(3H,s),2.20~3.30(4H,m),3.90~4.95(3H,m)

Example 20

Production of diphenylmethyl 2-[(4RS)-4-methylsulfonyl-3-oxo-2-isoxazolidinyl]-5-oxo-2-tetrahydrofuran carboxylate :

In 5 mℓ of methylene chloride was dissolved 180 mg of diphenylmethyl 2-[(4RS)-4-methylthio-3-oxo-2-isoxazolidinyl]-5-oxo-2-tetrahydrofuran carboxylate. To the solution was added 245 mg of m-chloroperbenzoic acid. The mixture was allowed to undergo reaction at room temperature for 2 hours. The reaction solution was washed with an aqueous solution of sodium hydrogen carbonate and then with an aqueous

solution of sodium thiosulfate, followed by drying over anhydrous sodium sulfate. The solvent was distilled off, and the residue was subjected to a silica-gel column chromatography, eluting with ethyl acetate - n-hexane (1:1) to give 164 mg of the above-titled compound. IR $\nu_{max}^{Nujol}$ $cm^{-1}$:

1780,1760,1700,1325,1180,1150
NMR(DMSO-$d_6$,ppm): 2.30~4.00(4H,m),3.20(3H,s),4.10~4.90(3H,m)

Example 21

Production of sodium 2-[(4RS)-4-methylsulfonyl-3-oxo-2-isoxazolidinyl]-5-oxo-2-tetrahydrofuran carboxylate :

To 150 mg of diphenylmethyl 2-[(4RS)-4-methylsulfonyl-3-oxo-2-isoxazolidinyl]-5-oxo-2-tetrahydrofuran carboxylate was added 2mℓ of 99% formic acid. The mixture was stirred at room temperature for one hour, followed by concentration under reduced pressure. The concentrate was washed with n-hexane, which was then neutralized by the addition of 2 mℓ of water and 70 mg of sodium hydrogen carbonate. The resultant was shaken with ethyl acetate, then the aqueous layer was subjected to a CHP-20 column chromatography, eluting with only water. The eluate was freeze-dried to give 65 mg of the above-titled compound as colorless powder. IR $\nu_{max}^{KBr}$ $cm^{-1}$:

1770,1710,1630,1370,1300,1190,1140
NMR($D_2O$,ppm external standard): 2.35~3.50(4H,m),3.30(3H,s),4.10~5.00(3H,m)

Example 22

Production of diphenylmethyl 2-[(4RS)-4-(2-hydroxy-2-propyl)-3-oxo-2-isoxazolidinyl]-5-oxo-2-tetrahydrofuran carboxylate :

In 70 mℓ of dry benzene was suspended 600 mg of diphenylmethyl 2-[(4RS)-4-(2-hydroxy-2-propyl)-4-isocyano-3-oxo-2-isoxazolidinyl]-5-oxo-2-tetrahydrofuran carboxylate. To the suspension were added 0.75 mℓ of tri-n-butyltin hydride and a catalytic amount of azobisisobutyronitrile. The mixture was heated under reflux for 30 minutes under argon stream. The solvent was distilled off under reduced pressure. The residue was subjected to a silica-gel column chromatography, eluting with methylene chloride - acetonitrile (9:1) to give 320 mg of the above-titled compound. IR $\nu_{max}^{Nujol}$ $cm^{-1}$:

1785,1765,1710,1175,1050,750,705
NMR($CDCl_3$,ppm): 1.30(6H,s),2.10~3.40(5H,m),4.10~4.60(2H,m),7.00(1H,s),7.20~7.50(10H,s)

Example 23

Production of sodium 2-[(4RS)-4-(2-hydroxy-2-propyl)-3-oxo-2-isoxazolidinyl]-5-oxo-2-tetrahydrofuran carboxylate :

In 0.5 mℓ of 99% formic acid was suspended 110 mg of diphenylmethyl 2-[(4RS)-4-(2-hydroxy-2-propyl)-3-oxo-2-isoxazolidinyl]-5-oxo-2-tetrahydrofuran carboxylate. The suspension was stirred at room temperature for 3 hours, followed by distilling off formic acid under reduced pressure. The residue was adjusted to pH 7.2 with an aqueous solution of sodium hydrogen carbonate, which was shaken with ethyl acetate. The aqueous layer was subjected to a CHP-20 column chromatography, eluting with water. The eluate was freeze-dried to give 45 mg of the above-titled compound. IR $\nu_{max}^{KBr}$ $cm^{-1}$:

1775,1720(s),1710(s),1650,1380,1195
NMR($D_2O$,ppm extarnal standard): 1.39(6H,s),2.20~3.30(4H,m),4.30~4.70(2H,m)

Example 24

Production of diphenylmethyl 2-(4-isopropyliden-3-oxo-2-isoxazolidinyl)-5-oxo-2-tetrahydrofuran carboxylate :

In 3 mℓ of methylene chloride was dissolved 236 mg of diphenylmethyl 2-[(4RS)-4-(2-hydroxy-2-propyl)-3-oxo-2-isoxazolidinyl]-5-oxo-2-tetrahydrofuran carboxylate. To the solution were added, under ice-cooling, 86 mg of pyridine and 95 mg of thionyl chloride. The mixture was stirred at the same temperature for 10 minutes. To the reaction solution was added ethyl acetate, which was washed with water, followed by drying over anhydrous sodium sulfate. The resultant was concentrated under reduced pressure, and the concentrate was subjected to a silica-gel column chromatography, eluting with ethyl acetate - n-hexane (1:1) to give 160 mg of the above-titled compound. IR $\nu_{max}^{Neat}$ $cm^{-1}$:

1790,1760,1710,1665,1175,1060,700

NMR($CDCl_3$,ppm):1.73(3H,s),2.20(3H,s),2.30~3.40(4H,m),4.70~5.10(2H,m),7.00(1H,s),7.20~7.50(10H,m)

Example 25

In 5 mℓ of tetrahydrofuran was dissolved 160 mg of diphenylmethyl 2-(4-isopropyliden-3-oxo-2-isoxazolidinyl)-5-oxo-2-tetrahydrofuran carboxylate. To the solution were added 2.5 mℓ of water containing 31 mg of sodium hydrogen carbonate and 160 mg of 5% palladium carbon. The mixture was stirred for one hour in hydrogen stream. The catalyst was filtered off, and the filtrate was shaken with ethyl acetate. The aqueous layer was freeze-dried to give 62 mg of the above-titled compound as colorless powder. IR $\nu_{max}^{KBr}$ $cm^{-1}$:

1770,1710(s),1650,1380,1190,910

NMR($D_2O$,ppm extarnal standard): 1.86(3H,s),2.23(3H,s),2.20~3.30(4H,m)

Example 26

Production of diphenylmethyl 2-(3-oxo-2-isoxazolidinyl)-5-oxo-2-tetrahydrofuran carboxylate :

In 20 mℓ of dry benzene was dissolved 203 mg of diphenylmethyl 2-[(4S)-4-isocyano-3-oxo-2-isoxazolidinyl]-5-oxo-2-tetrahydrofuran carboxylate. To the solution were added 0.25 mℓ of tri-n-butyltin hydride and a catalytic amount of azobisisobutyronitrile. The mixture was heated under reflux for 30 minutes in argon stream. The solvent was distilled off under reduced pressure. The residue was subjected to a silica-gel column chromatography, eluting with ethyl acetate - n-hexane (1:1) to give 180 mg of the above-titled compound. IR $\nu_{max}^{Nujol}$ $cm^{-1}$:

1785,1760,1720,1180,1060

NMR(DMSO-$d_6$,ppm): 2.10~3.30(6H,m),4.33(2H,t,J=9Hz),6.90(1H,s),7.20~7.50(10H,m)

Example 27

Production of sodium 2-(3-oxo-2-isoxazolidinyl)-5-oxo-2-tetrahydrofuran carboxylate :

In 12 mℓ of tetrahydrofuran was dissolved 180 mg of diphenylmethyl 2-(3-oxo-2-isoxazolidinyl)-5-oxo-2-tetrahydrofuran carboxylate. To the solution were added 40 mg of sodium hydrogen carbonate, 180 mg of 5% palladium carbon and 6 mℓ of water. The mixture was stirred for one hour in hydrogen stream. The catalyst was filtered off, and the filtrate was concentrated under reduced pressure. The concentrate was subjected to a CHP-20 column chromatography, eluting with water. The eluate was freeze-dried to give 60 mg of the above-titled compound. IR $\nu_{max}^{KBr}$ $cm^{-1}$:

1780,1710,1650,1390,1195,
NMR($D_2O$,ppm external standard): 2.30~3.30(6H,m),4.46(2H,t,J=9Hz)

Example 28

Production of diphenylmethyl 2-[(4RS)-4-(2-hydroxyethyl)-4-isocyano)-3-oxo-2-isoxazolidinyl]-5-oxo-2-tetrahydrofuran carboxylate :

In 4 mℓ of dimethylformamide was dissolved 406 mg of diphenylmethyl 2-[(4S)-4-isocyano-3-oxo-2-isoxazolidinyl]-5-oxo-2-tetrahydrofuran carboxylate. To the solution were added, under ice-cooling, 0.1 mℓ of 80% acetaldehyde and 138 mg of pulverized anhydrous potassium carbonate. The mixture was stirred at the same temperature for 30 minutes. To the resultant were added ethyl acetate and water, and the ethyl acetate layer was washed with water, dried over anhydrous sodium sulfate and concentrated under reduced pressure. The concentrate was subjected to a silica-gel column chromatography, eluting with ethyl acetate : n-hexane (1:1) to give 280 mg of the above-titled compound. IR $\nu_{max}^{Nujol}$ $cm^{-1}$:

2120,1790,1760,1720,1380,1060
NMR($CDCl_3$,ppm): 1.36(3H,d,J=7Hz),1.60(1H,br.s),2.20~3.50(4H,m),4.00~4.80(3H,m),7.00(1H,s),7.20~7.50-(10H,br.s)

Example 29

Production of diphenylmethyl 2-[(4RS)-4-(2-hydroxyethyl)-3-oxo-2-isoxazolidinyl]-5-oxo-2-tetrahydrofuran carboxylate:

In 5 mℓ of dry benzene was dissolved 200 mg of diphenylmethyl 2-[(4RS)-4-(2-hydroxyethyl)-4-isocyano-3-oxo-2-isoxazolidinyl]-5-oxo-2-tetrahydrofuran carboxylate. To the solution were added 0.15 mℓ of tri-n-butyltin hydride and a catalytic amount of azobisisobutyronitrile. The mixture was heated under reflux for 30 minutes in argon stream, which was concentrated under reduced pressure. The concentrate was washed with n-hexane. The residual insolubles were subjected to a silica-gel column chromatography, eluting with ethyl acetate - n-hexane (1:1) to give 170 mg of the above-titled compound. IR $\nu_{max}^{Nujol}$ $cm^{-1}$:

1790,1760,1720,1180,1060,750,705
NMR($CDCl_3$,ppm): 1.23(3H,d,J=7Hz),1.66(1H,br.s),2.10~3.50(4H,m),3.80~4.90(3H,m),6.96(1H,s),7.20~7.50-(10H,m)

Example 30

Production of sodium 2-{4-[2-(2-amino-4-thiazolyl)-(Z)-2-(methoxyimino)-acetamido]-3-oxo-2-isoxazolidinyl}-5-oxo-2-tetrahydrofuran carboxylate [Compound (30c)] :

(a) Production of diphenylmethyl 2-{4-[2-(2-chloroacetamido-4-thiazolyl)-(Z)-2-(methoxyimino)acetamido]-3-oxo-2-isoxazolidinyl}-5-oxo-2-tetrahydrofuran carboxylate [Compound (30a)] :

In 4 mℓ of dry methylene chloride was dissolved 210 mg of 2-{(4RS)-4-[2-(2-chloroacetamido-4-thiazolyl)-(Z)-2-(methoxyimino)acetamido]-4-methylthio-3-oxo-2-isoxazolidinyl}-5-oxo-2-tetrahydrofuran carboxylate. To the solution was added 145 mg of m-chloroperbenzoic acid, and the reaction was allowed to proceed at room temperature for 20 hours. The reaction solution was concentrated under reduced pressure, and the concentrate was dissolved in ethyl acetate. The solution was washed with an aqueous solution of sodium hydrogen carbonate, dried over anhydrous magnesium sulfate and concentrated under reduced

pressure. The concentrate was subjected to a silica-gel column chromatography, eluting with ethyl acetate - n-hexane (1:1) to give 162 mg of the above-titled Compound (30a). IR $\nu_{max}^{KBr}$ $cm^{-1}$:

1815,1765,1710(s),1675,1640,1540,1260,1170,1040,785,755,695
NMR($CDCl_3$,ppm): 2.20~3.00(3H,m),3.36~3.80(1H,m)4.00(3H,s),4.16(2H,s),7.00(1H,s),7.20~7.50(11H,m),8.60-(1H,s),9.30(1H, br.s)

(b) Produciton of diphenylmethyl 2-{4-[2-(2-amino-4-thiazolyl-(Z)-2-(methoxyimino)acetamido]-3-oxo-2-isoxazolidinyl}-5-oxo-2-furan carboxylate [Compound (30b)] :

In 2 mℓ of dimethylformamide was dissolved 160 mg of the Compound (30a) obtained as above. To the solution was added, under ice-cooling, 41 mg of sodium N-methyldithiocarbamate. The reaction was allowed to proceed at the same temperature for 2.5 hours. The reaction solution was poured into water, which was subjected to extraction with ethyl acetate. The extract was washed with water, dried over anhydrous magnesium sulfate and concentrated under reduced pressure. The concentrate was subjected to a silica-gel column chromatography, eluting with ethyl acetate - n-hexane (1:1) to give 79 mg of the above-titled Compound (30b). IR $\nu_{max}^{KBr}$ $cm^{-1}$:

1820,1770,1710,1680,1645,1535,1250,1180,1050,700
NMR($CDCl_3$,ppm): 2.20~3.10(3H,m),3.30~3.80(1H,m),3.93(3H,s),5.63(2H,br.s),6.73(1H,s),6.96(1H,s)-,7.20~7.50(10H,m),8.70(1H,s),9.43(1H,br.s)

(c) Production of sodium 2-4-[2-(2-amino-4-thiazolyl)-(Z)-2-(methoxyimino)acetamido]-3-oxo-2-isoxazolidinyl -5-oxo-2-furan carboxylate [Compound (30c)] :

In 1 mℓ of formic acid was dissolved, under ice-cooling, 79 mg of the Compound (30b) obtained as above. The solution was stirred at room temperature for one hour. Formic acid was distilled off under reduced pressure. The residue was adjusted to pH 7 by the addition of an aqueous solution of sodium hydrogen carbonate, which was subjected to a CHP-20 column chromatography, eluting with 10% ethanol-water. The eluate was freeze-dried to give 43 mg of the above-titled Compound (30c). IR $\nu_{max}^{KBr}$ $cm^{-1}$:

1780,1700(s),1680(s),1660,1635,1525,1380,1030
NMR($D_2O$,ppm,extarnal standard): 2.10~3.20(3H,m),3.20~3.70(1H,m),3.80(3H,s),7.20(1H,s),8.60(1H,s)

**Claims**

1. A compound of the formula:

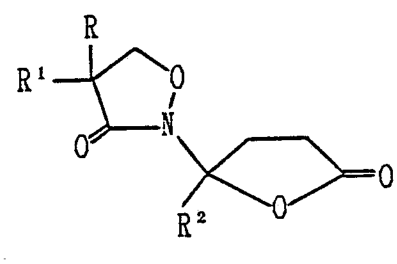

wherein $R^1$ is hydrogen, isonitrile or an organic residue bonded through nitrogen; R is hydrogen, alkyl which may be substituted, alkylthio in which sulfur atom may be oxidized or azido, or forms a double bond together with the adjacent carbon atom; provided that when $R^1$ is an organic residue bonded through nitrogen, R is not hydrogen; and $R^2$ is carboxyl or a group derivable therefrom, or a salt thereof.

2. The compound according to claim 1, wherein $R^1$ is isonitrile.

3. The compound according to claim 1, wherein $R^1$ is an organic residue bonded through nitrogen.

4. The compound according to claim 3, wherein the organic residue bonded through nitrogen is acylamino, amino substituted through carbon, alkenylamino, thioamino, silylamino, phosphoamino or a group represented by the formula: -CO-CO-NH-.

5. The compound according to claim 4, wherein the acylamino is a group represented by the formula: $R^8$-$R^9$-CONH-wherein $R^8$ is of the formula $R^{10}$-C(=N-$OR^{11}$)-; or of the formula -$R^{12}$-$R^{13}$, $R^9$ is a bond or of the formula -CONH-$CHR^{14}$-.

6. The compound according to claim 5, wherein acylamino is 2-(2-amino-4-thiazolyl)-2-(methoxyimino)-acetamido.

7. The compound according to claim 1, wherein R is alkyl which may be substituted.

8. The compound according to claim 7, wherein R is straight-chain or branched alkyl of 1 to 6 carbon atoms which may be substituted with hydroxyl, substituted hydroxycarbonyl, substituted carbonyloxy, $C_{1-4}$ alkyl sulfonyloxy or azido.

9. The compound according to claim 1, wherein R is alkylthio of 1 to 4 carbon atoms in which the sulfur atom may have 1 or 2 oxygen atoms.

10. The compound according to claim 1, wherein R forms a double bond together with the adjacent carbon atom on the isoxazole ring to form an endo double bond, or on the substituent thereof to form an exo double bond.

11. The compound according to claim 1, wherein $R^1$ and R are each hydrogen atom.

12. The compound according to claim 1, wherein $R^2$ is carboxyl.

13. The compound according to claim 1, wherein $R^2$ is a group represented by the formula: -$COOR^{37}$ wherein $R^{37}$ is alkyl, alkenyl, aryl, cycloalkyl, heterocyclic ring or silyl which may be substituted; or the formula: -$CONR^{38}R^{39}$ wherein $R^{38}$ and $R^{39}$ are independently hydrogen, alkyl, aryl, cycloalkyl, alkenyl or heterocyclic ring which may be substituted, or form a heterocyclic ring together with the adjacent nitrogen atom.

14. The compound according to claim 1, which is sodium 2-{(4RS)-4-[2-(2-amino-4-thiazolyl)-(Z)-2-(methoxyimino)acetamido]-4-hydroxymethyl-3-oxo-2-isoxazolidinyl}-5-oxo-2-tetrahydrofuran carboxylate.

15. The compound according to claim 1, which is sodium 2-{(4RS)-4-[2-(2-amino-4-thiazolyl)-(Z)-2-(methoxyimino)acetamido]-4-acetoxymethyl-3-oxo-2-isoxazolidinyl}-5-oxo-2-tetrahydrofuran carboxylate.

16. The compound according to claim 1, which is sodium 2-{(4RS)-4-[2-(2-amino-4-thiazolyl)-(Z)-2-(methoxyimino)acetamido]-4-carbamoyloxymethyl-3-oxo-2-isoxazolidinyl}-5-oxo-2-tetrahydrofuran carboxylate.

17. A method for producing the compound according to claim 1, which comprises reacting a compound of the formula :

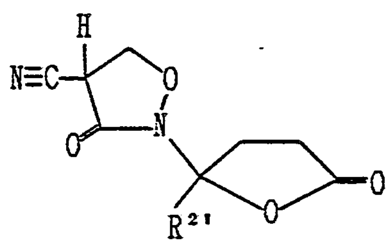

wherein $R^{2'}$ is a group derivable from carboxyl, with a nucleophilic reagent, and if desired, subjecting the resulting compound to hydrolysis, elemination reaction, substitution reaction and/or converting reaction.

European Patent Office

# EUROPEAN SEARCH REPORT

Application number

EP 87 30 4458

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CH-A- 321 326 (MERCK & CO. INC.)<br>* page 1, line 1 - page 2, line 2 *<br>--- | 1 | C 07 D 413/04<br>C 07 K 5/06<br>A 61 K 31/42<br>A 61 K 37/02 |
| A | CHEMICAL ABSTRACTS, vol. 82, no. 11, 17th March 1975, page 485, column 2, abstract nr. 73431u, Columbus, Ohio, US; R.B. MORIN et al.: "Chemistry of dehydropeptides. Rearrangement of peptide isoxazolidinones derived from cycloserine", & TETRAHEDRON. LETT. 1974, (34), 2979-2982<br>--- | 1 | |
| A | DE-A-2 436 960 (MERCK & CO. INC.)<br>* claim 9 *<br>--- | 1 | TECHNICAL FIELDS SEARCHED (Int. Cl.4) |
| A | CHEMICAL ABSTRACTS, vol. 88, no. 25, 19th June 1978, page 824, column 1, abstract no. 191406m, Columbus, Ohio, US; A. TSUJI et al.: "Reactive D-alanyl-D-alanine peptide derived from cycloserine", & HETEROCYCLES 1977, 8, 153-157 (Cat. D)<br>--- | 1 | C 07 D 413/00<br>C 07 K 5/00<br>A 61 K 31/00<br>A 61 K 37/00<br>C 07 D 417/00 |
| A | DE-A-2 728 766 (FUJISAWA PHARMACEUTICAL CO. LTD.)<br>--- -/- | 1 | |

The present search report has been drawn up for all claims

| Place of search | Date of completion of the search | Examiner |
|---|---|---|
| BERLIN | 31-08-1987 | VAN AMSTERDAM L.J.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

European Patent Office

# EUROPEAN SEARCH REPORT

Application number

EP 87 30 4458

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | NATURE, vol. 325, no. 7000, 8th-14th January 1987, pages 179-180; Y. NOZAKI et al.: "Binding of a non-beta-lactam antibiotic to penicillin-binding proteins" * page 180, figure 1 * | 1 | |
| | --- | | |
| P,A D | EP-A-0 191 989 (TAKEDA CHEMICAL INDUSTRIES LTD.) * claims * | 1,5,6 | |
| | ----- | | |

TECHNICAL FIELDS SEARCHED (Int. Cl.4)

The present search report has been drawn up for all claims

| Place of search | Date of completion of the search | Examiner |
|---|---|---|
| BERLIN | 31-08-1987 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document